# EUROPÄISCHE PATENTSCHRIFT

(11) **EP 3 273 938 B1**
(45) Veröffentlichungstag und Bekanntmachung des Hinweises auf die Patenterteilung: **28.10.2020**
(21) Anmeldenummer: 16721347.9
(22) Anmeldetag: 10.03.2016
(51) Int. Cl.: A61K 8/55, A61K 8/92, A61K 8/34, A61K 8/02, A61Q 19/00

(54) **KOSMETISCHES PRODUKT SOWIE KONZENTRAT ZUR HERSTELLUNG DES KOSMETISCHEN PRODUKTES**
COSMETIC PRODUCT AND CONCENTRATE FOR PRODUCING THE COSMETIC PRODUCT
PRODUIT COSMÉTIQUE ET CONCENTRÉ DESTINÉ À LA PRODUCTION DU PRODUIT COSMÉTIQUE

(30) Priorität: 25.03.2015 DE 102015003841
(43) Veröffentlichungstag der Anmeldung: 31.01.2018
(73) Patentinhaber: GCT GmbH, 42799 Leichlingen (DE)
(72) Erfinder: ALBRECHT, Martin, 51519 Odenthal (DE); KOMP, Bernd, 2587 BB The Hague (NL)
(74) Vertreter: Simandi, Claus
(86) Internationale Anmeldenummer: PCT/DE2016/000107
(87) Internationale Veröffentlichungsnummer: WO 2016/150417

(56) Entgegenhaltungen:
- WO-A2-02/089770
- FR-A1- 2 904 766
- JP-A- 2012 051 872
- US-A- 6 117 434
- US-A1- 2014 364 418
- G. PENNICK ET AL: "Superior effect of isostearyl isostearate on improvement in stratum corneum water permeability barrier function as examined by the plastic occlusion stress test", INTERNATIONAL JOURNAL OF COSMETIC SCIENCE., Bd. 32, Nr. 4, 20. April 2010 (2010-04-20) , Seiten 304-312, XP055281991, NL ISSN: 0142-5463, DOI: 10.1111/j.1468-2494.2010.00604.x
- ANONYMOUS: "Amended final report on the safety assessment of Oryza Sativa (Rice) Bran Oil, Oryza Sativa (Rice) Germ Oil, Rice Bran Acid, Oryza Sativa (Rice) Bran Wax, Hydrogenated Rice Bran Wax, Oryza Sativa (Rice) Bran Extract, Oryza Sativa (Rice) Extract, Oryza Sativa (Rice) Germ Powder, Oryza Sativa (Rice) S", INTERNATIONAL JOURNAL OF TOXICOLOGY,, Bd. 25, Nr. Suppl. 2, 1. März 2006 (2006-03-01), Seiten 91-120, XP008117683, ISSN: 1091-5818, DOI: 10.1080/10915810600964626

## Beschreibung

Die vorliegende Erfindung betrifft ein kosmetisches Produkt mit den Merkmalen des Oberbegriffs des Patentanspruchs 1 sowie ein Konzentrat zur Herstellung eines derartigen kosmetischen Produktes.

Ein kosmetisches Produkt mit den Merkmalen des Oberbegriffs des Patentanspruchs 1 ist aus der WO 2009/043341 bekannt. Hierbei weist die bekannte Zusammensetzung als Inhaltsstoffe ein hydriertes Phospholipid in einer Konzentration von wenigstens 0,7 Gew.%, mindestens einen zweiwertigen und/oder dreiwertigen Alkohol sowie mindestens ein Wachs auf, wobei nur im Ausführungsbeispiel M die Konzentration des Wachses, bei dem es sich um Reiskleiewachs handelt, mit 0,1 Gew.% quantifiziert ist, während in diesem Ausführungsbeispiel die Konzentration des hydrierten Phosphatidylcholins mit 1,5 Gew.% angegeben ist. Hieraus errechnet sich ein Verhältnis von hydriertem Phospholipid zu Wachs zu 1:0,066. Hauptsächlich jedoch stellt die WO 2009/043341 darauf ab, daß die bekannte Formulierung lamellare Strukturen aufweist, die sandwichartig übereinander angeordnete lamellare Doppelmembranschichten umfaßt. Zwischen benachbarten, parallel zueinander ausgerichtete Doppelmembranschichten ist jeweils eine Schicht einer inneren wäßrigen Phase vorgesehen. Die Wirkstoffe sind dabei unabhängig davon, ob sie lipophil oder hydrophil sind, jeweils in den Doppelmembranen als auch in den inneren wäßrigen Phasen angeordnet, jedoch in unterschiedlichen Konzentrationen, während die äußere wäßrige Phase, die die lamellare Struktur umgibt, weitestgehend frei von Wirkstoffen ist.

Die WO 02/089770 ist ausschließlich auf eine pharmazeutische Zusammensetzung und somit auf ein Arzneimittel gerichtet und enthält kein pflanzliches Wachs.

Die US 6,117,437 beschreibt im Beispiel 29 zwar ein Gewichtsverhältnis von hydriertem Phospholipid zu Wachs von 1:1, jedoch ist dieses Wachs **kein** pflanzliches Wachs sondern ein "microcrystalline wax". Ein "microcrystalline wax" wird im deutschen Sprachraum als "Mikrowachs" bezeichnet, gehört jedoch nicht zu pflanzlichen Wachsen sondern zu den Mineralwachsen.

Der vorliegenden Erfindung liegt die Aufgabe zugrunde, ein kosmetisches Produkt der angegebenen Art zur Verfügung zu stellen, das eine besonders hohe Wirksamkeit in bezug auf die Verhinderung oder Beseitigung von Barrierestörungen der Haut aufweist.

Desweiteren liegt der vorliegenden Erfindung die Aufgabe zugrunde, ein Konzentrat zur Verfügung zu stellen, aus dem durch Verdünnen mit einem wäßrigen System das kosmetische Produkt besonders einfach und schnell herstellbar ist.

Diese Aufgaben werden erfindungsgemäß durch ein kosmetisches Produkt mit den kennzeichnenden Merkmalen des Patentanspruchs 1 sowie durch ein Konzentrat mit den Merkmalen des Patentanspruchs 20 gelöst. Erfindungsgemäß wird somit ein kosmetisches Produkt vorgeschlagen, das wie die zuvor beschriebene bekannte Formulierung neben Wasser mindestens ein hydriertes Phospholipid in einer Konzentration von wenigstens 0,7 Gew.%, mindestens einen zweiwertigen und/oder dreiwertigen Alkohol sowie mindestens ein pflanzliches Wachs enthält. Im Unterschied zu der bekannten, vorstehend beschriebenen Formulierung ist jedoch bei dem erfindungsgemäßen kosmetischen Produkt das Gewichtsverhältnis von hydrierten Phospholipid zu dem Wachs dahingehend quantifiziert, daß in dem erfindungsgemäßen kosmetischen Produkt das Gewichtsverhältnis von hydrierten Phospholipid zu Wachs zwischen 1:0,3 und 1:1,5, insbesondere zwischen 1:0,7 und 1:1,1, variiert. Desweiteren liegt in dem

erfindungsgemäßen kosmetischen Produkt das hydrierte Phospholipid zumindest teilweise in einer ortho rhombischen lamellaren kristallinen Struktur vor, wobei diese ortho rhombische lamellare kristalline Struktur nachfolgend noch genau erklärt wird. In der ortho rhombischen lamellaren kristallinen Struktur ist das Wachs eingelagert und/oder an der ortho rhombischen lamellaren kristallinen Struktur angelagert.

Überraschend wurde festgestellt, daß die zuvor beschriebene bekannte Zusammensetzung zwar auch eine lamellare Struktur auf, jedoch handelt es sich bei dieser lamellaren Struktur um eine hexagonale lamellare Struktur und nicht, wie erfindungsgemäß vorgeschlagen, um eine ortho rhombische lamellare kristalline Struktur. Weiterhin wird als erfindungswesentlich angesehen, daß sich die erwünschte, in dem erfindungsgemäßen kosmetischen Produkt enthaltene ortho rhombische lamellare kristalline Struktur nur dann ausbildet, wenn das Gewichtsverhältnis von hydrierten Phospholipid zu dem Wachs zwischen 1:0,3 und 1:1,5, insbesondere zwischen 1:0,7 und 1:1,1, variiert. Wird dieses Gewichtsverhältnis unterschritten oder überschritten, so bilden sich aus dem hydrierten Phospholipid und dem darin eingelagerten und/oder daran angelagerten Wachs liquide lamellare Strukturen oder hexagonal lamellare Strukturen, jedoch keine ortho rhombische lamellaren kristallinen Strukturen aus, wie dies nachfolgend bei den Ausführungsbeispielen noch beschrieben und ausführlich diskutiert ist.

Gerade diese ortho rhombischen lamellaren kristallinen Strukturen, die ausschließlich bei den zuvor quantifizierten Gewichtsverhältnissen von hydriertem Phospholipid zu dem Wachs ausgebildet werden, sind in der Lage, Barrierestörungen der Haut zu verhindern und desweiteren vorhandene Barrierestörungen der Haut besonders wirksam zu beseitigen, so daß das erfindungsgemäße kosmetische Produkt nicht nur prophylaktisch sondern auch therapeutisch zur Behandlung von Barrierestörungen der Haut hervorragend verwendbar ist, wie dies nachfolgend noch anhand bei den Ausführungsbeispielen beschriebenen Wirksamkeitsstudien belegt wird.

Die zuvor beschriebene verbesserte prophylaktische und therapeutische kosmetische Wirksamkeit des erfindungsgemäßen kosmetischen Produktes wird darauf zurück, daß das erfindungsgemäße kosmetische Produkt besonders effektiv das Auftreten von Fehlstellen oder das Vorhandensein von Fehlstellen in den Interzellularlipiden der Hornhaut verhindert bzw. beseitigt, wodurch der transepidermale Wasserverlust, der ein Maß für die zuvor angesprochenen Barrierestörungen ist, in einem Bereich gehalten oder in einen Bereich zurückgeführt wird, der der gesunden, nicht geschädigten Haut entspricht. Aufgrund der starreren, eine geringe Beweglichkeit der Moleküle bewirkenden und des kompakteren Aufbaus der ortho rhombischen lamellaren kristallinen Struktur im Vergleich zu einer hexagonal lamellaren Struktur ist das erfindungsgemäße kosmetische Produkt wesentlich besser in der Lage, die zuvor angesprochene Barrierestörung und insbesondere Störstellen in den Interzellularlipiden besser, schneller und wirksamer zu beseitigen oder zu verhindern als eine hexagonal lamellare Struktur oder eine liquide lamellare Struktur. Auch dringt das erfindungsgemäße Produkt nicht so tief in die unteren Hautschichten ein sondern verbleibt vielmehr hauptsächlich in der Hornschicht und stellt hier sicher, daß die Interzellularlipide, die zwischen den verhornenden mehrschichtigen Plattenepithelen bestehen, keine oder weniger Fehlstellen ausbilden oder die dort vorhandenen Fehlstellen durch die ortho rhombische lamellare kristalline Struktur ausgebessert werden.

Als hydriertes Phospholipid, das in dem erfindungsgemäßen kosmetischen Produkt enthalten ist, ist grundsätzlich jedes hydrierte Phospholipid geeignet, das mit dem Wachs in dem zuvor beschriebenen Gewichtsverhältnissen ortho rhombische lamellare kristalline Strukturen aufbaut. Insbesondere werden in dem erfindungsgemäßen kosmetischen Produkt solche hydrierten Phospholipide vorgesehen, die aus der Gruppe ausgewählt sind, die hydriertes Phosphatidylethanolamin, hydriertes Phosphatidylinositol, hydriertes Phosphatidylcholin, hydriertes Lyso-Phosphatidylcholin, hydriertes Phosphatidylserin und hydrierte Phosphatidsäure umfaßt.

Besonders gute Ergebnisse bezüglich der Verhinderung und/oder der Beseitigung von Barrierestörungen und insbesondere von Fehlstellen in den Interzellularlipiden und damit zur Erhaltung einer gesunden Haut und/oder zur Behandlung von geschädigter Haut weisen solche Ausführungsformen des erfindungsgemäßen kosmetischen Produktes auf, bei denen das hydrierte Phospholipid aus pflanzlichen Phospholipiden, vorzugsweise aus Sojalecithin oder Sonnenblumenlecithin, durch Hydrierung hergestellt ist und eine Konzentration an hydriertem Phosphatidylcholin von wenigstens 60 Gew.%, bezogen auf das eingesetzte hydrierte Phospholipid, aufweist. Die prophylaktischen und therapeutische Wirksamkeit des erfindungsgemäßen kosmetischen Produktes wird noch dadurch weiter verbessert, daß ein hydriertes Phospholipid mit einer Konzentration an hydriertem Phosphatidylcholin zwischen 70 Gew.% und 85 Gew.% und insbesondere zwischen 90 Gew.% und 98 Gew.% verwendet wird.

Bezüglich des Wachses, das in dem erfindungsgemäßen kosmetischen Produkt enthalten ist und das mit dem hydrierten Phospholipid in den zuvor angesprochenen Gewichtsverhältnissen die ortho rhombische lamellare kristalline Struktur ausbildet, wird ein pflanzliches Wachs ausgewählt, das aus Blättern, Nadeln, Stengeln, Wurzeln, Rinden, Kleie , Schalen, Samen, Blüten und/oder Früchten isoliert ist. Hierzu gehören insbesondere solche pflanzlichen Wachse (allein oder in Mischung), die aus der Gruppe ausgewählt sind, die Carnaubawachs, Candelillawachs, Ouricuriwachs, Zuckerrohrwachs, Retamowachs, Carandaywachs, Raffiawachs, Columbiawachs, Espartowachs, Alfalfawachs, Bambuswachs, Hanfwachs, Douglas Fir-Wachs, Korkwachs, Sisalwachs, Flachswachs, Baumwollwachs, Dammarwachs, Getreidewachs, Teewachs, Kaffeewachs, Ocatillawachs, Citrus Aurantium Dulcis Schalenwachs, Ficus Ceriferawachs, Orangenwachs, Sonnenblumenkernwachs, Sonnenblumenkernschalenwachs, Sprossenkohlwachs, Tabakpflanzenwachs, Kürbiskernwachs, Maiswachs, Feigenkaktus Wachs und Oleanderwachs umfaßt.

Eine besonders geeignete hochwirksame Ausgestaltung des erfindungsgemäßen kosmetischen Produktes weist als Wachs insbesondere das Carnaubawachs, Sonnenblumenkernwachs, Reiswachs oder das Reiskleiewachs allein oder in Mischung untereinander oder in Mischung mit den zuvor beschriebenen Wachsen auf. Insbesondere dann, wenn das Wachs und vorzugsweise das Carnaubawachs, Sonnenblumenkernwachs, Reiswachs und/oder das Reiskleiewachs, die chemisch als Ester anzusprechen sind, als Hauptfettsäurekomponente gesättigte C₂₂-C₂₆-Fettsäuren enthält, werden die eingangs angesprochenen Vorteile des erfindungsgemäßen kosmetischen Produktes im besonders hohem Maße erreicht, wobei es besonders bevorzugt ist, wenn das verwendete Wachs und vorzugsweise das Carnaubawachs, Sonnenblumenkernwachs, Reiswachs und/oder das Reiskleiewachs diese Hauptfettsäurekomponente in einer Konzentration zwischen 15 Gew.% und 33 Gew.%, insbesondere zwischen 23 Gew.% und 28 Gew.%, bezogen auf das Gewicht des jeweiligen Wachses, enthält.

Eine andere, besonders bevorzugte Ausgestaltung des erfindungsgemäßen kosmetischen Produktes schlägt vor, daß hierbei das Wachs und vorzugsweise das Carnaubawachs, Sonnenblumenkernwachs, Reiswachs oder das Reiskleiewachs zusätzlich noch freie lineare C₃₀-C₃₄-Alkohole und/oder freie C₁₆-C₂₄-Fettsäuren enthält, insbesondere in einer Fettsäurekonzentration zwischen 1 Gew.% und 16 Gew.%, bezogen auf das Gewicht des jeweiligen Wachses.

Um die Lagerfähigkeit des erfindungsgemäßen kosmetischen Produktes zu verbessern, ist es besonders vorteilhaft, die Konzentration der ungesättigten Fettsäuren in den zuvor beschriebenen freien C₁₆-C₂₄-Fettsäuren zu begrenzen, wobei insbesondere die Konzentration der ungesättigten Fettsäuren in den freien Fettsäuren zwischen 0,05 Gew.% und 0,4 Gew.%, bezogen auf das Gewicht des jeweiligen Wachses oder der Wachsmischung und insbesondere bezogen auf das Gewicht des Carnaubawachses, Sonnenblumenkernwachses, Reiswachses und/oder des Reiskleiewachses, beträgt oder eingestellt wird.

Desweiteren sieht eine andere Ausführungsform des erfindungsgemäßen kosmetischen Produktes zur Verlängerung der Lagerstabilität und zur Verhinderung einer unerwünschten Oxidation vor, daß hierbei das erfindungsgemäße kosmetische Produkt mindestens ein Antioxidationsmittel aufweist. Vorzugsweise wird dieses Antioxidationsmittel aus der Gruppe ausgewählt, die Tocopherole, Polyphenole, Epigallocatechine, Epigallocatechingallat Kaffeesäure, Flavonoide, Ellaginsäure, Curcuminderivate, Dihydroquercetin, Tetrahydrocurcuminoid, Tetrahydrodiferuloylmethane, L-Carnosin, N-Acetylcystein, Phytinsäure, Chelatbildner, so insbesondere Thioctinsäure und/oder EDTA, BHA, BHT, pflanzliche Inhaltsstoffe, wie Picea abies extract, Pycnogenol, Bakuchiol, Hydroxityrosol, und Derivate, Bis-Ethylhexyl Hydroxydimethoxy Benzylmalonat (Ronacare AP; Hersteller Merck) Lipochromane, insbesondere Lipochroman-6, umfaßt. Abhängig von dem jeweiligen Antioxidationsmittel variiert dessen Konzentration bevorzugt zwischen 0,01 Gew.% und 10 Gew.%, bezogen auf das anwendungsfertige kosmetische Produkt.

Wie bereits zuvor bei dem erfindungsgemäßen kosmetischen Produkt ausgeführt ist, enthält das kosmetische Produkt einen zweiwertigen und/oder dreiwertigen Alkohol, wobei es sich hierbei vorzugsweise um ein Diol und/oder um Glycerin handelt. Besonders bevorzugte Alkohole sind neben Glycerin, Pentylenglycol, insbesondere 1,2-Pentandiol, Hexylenglycol, insbesondere 1,2-Hexandiol, Octandiol und/oder Butylcyclohexanol, vorzugsweise tertiäres Butylcyclohexanol und insbesondere 4-t-Butylcyclohexanol, jeweils allein oder in beliebiger Mischung. Insbesondere die zuvor aufgeführten Alkohole besitzen eine besonders hohe Hautverträglichkeit und fördern die Bildung der ortho rhombischen lamellaren kristallinen Struktur.

Eine besonders bevorzugte Ausführungsform des erfindungsgemäßen kosmetischen Produktes sieht vor, daß das kosmetische Produkt als weiteren Inhaltsstoff Isostearylisostearat in einer Konzentration zwischen 1 Gew.% und 20 Gew.% enthält. Hierdurch wird insbesondere erreicht, daß das kosmetische Produkt eine hautpflegende Eigenschaft besitzt.

Das zuvor bei den Alkoholen bereits beschriebene Butylcyclohexanol und insbesondere die tertiären Butylcyclohexanole und vorzugsweise das 4-t-Butylcyclohexanol sind nicht nur bevorzugte Alkohole sondern auch ausgezeichnete Wirkstoffe, das sie zu der Gruppe der TRPV1-Inhibitoren (TRPV1_= Transient receptor potential channel, vanilloid subfamily member 1) gehören, die die Reizschwelle der Haut herabsetzen und überschießenden Hautreaktionen, welche u.a. bei empfindlichen, barrieregestörten Hautzuständen auftreten, entgegenwirken.

Bezüglich der Konzentration an hydrierten Phosphatidylcholin in dem erfindungsgemäßen kosmetischen Produkt ist festzuhalten, daß diese Konzentration insbesondere zwischen 0,7 Gew.% und 8 Gew.%, vorzugsweise zwischen 1,2 Gew.% und 5 Gew.%, variiert.

Abhängig von dem jeweiligen Verwendungszweck weist das erfindungsgemäße kosmetische Produkt desweiteren einen Lichtschutzfilter, einen UV-Filter, einen hautschützenden Wirkstoff, einen hautpflegenden Wirkstoff, einen glättenden Wirkstoff, einen die Haut geschmeidigmachenden Wirkstoff, einen hautaufhellenden Wirkstoff, einen bräunenden Wirkstoff, einen desodorierenden Wirkstoff, einen enthaarenden Wirkstoff, einen feuchthaltenden Wirkstoff, einen Wirkstoff zur Pflege und Behandlung von überempfindlicher Haut, einen Wirkstoff zur Behandlung und Pflege infizierter, gereizter oder erkrankter Haut, einen Wirkstoff zur Prophylaxe gegen Insektenstiche, einen rückfettenden Wirkstoff, einen antientzündlichen Wirkstoff und/oder einen feuchtigkeitsspendenden Wirkstoff auf.

Bevorzugte Lichtschutzfilter und UV-Filter sind insbesondere Benzophenon-3, Benzophenon-4, Benzophenon-5, 3-Benzyliden Campher, BenzylidenCampher Sulfonsäure, Butyl Methoxydibenzoylmethan, Campher Benzalkonium Methosulfat, Diethylhexyl Butamido Triazon (DiethylhexylButamido Triazone), Ethylhexyl Dimethyl PABA, Ethylhexyl Methixycinnamat, Ethylhexyl Salicylat, Ethylhexyl Triazon, Homosalat, Isoamyl p-Methoxycinnamat, 4-Methylbenzyliden Campher, Octocrylen, PABA (p-Aminobenzoesäure), PEG-25 PABA, Phenylbenzimidazol Sulfonsäure, Polyacrylamidomethyl Benzyliden Campher, Kalium Phenylbenzimidazol, Natrium Phenylbenzimidazolsulfonat, TEA-Phenylbenzimidazole Sulfonate (Tea-Phenylbenzimidazole Sulfonate) und Terephthalyliden-dicamphersulfonsäure (Terephthalylidene Dicamphor sulfonic acid), Oxybenzon, BEMT, Octocrylen, Benzophenon-9, Diethylamino-hydroxybenzoylhexylbenzoat, Drometrizole Trisiloxan, 4-Methylbenzylidene Campher, 3-Benzylidene Campher, Octylsalicylat, Methylene Bis-Benzotriazolyl Tetramethylbutylphenol, Bis-Ethylhexyloxyphenol Methoxyphenyl Triazin, Ethylhexylmethoxycinnamate, Diethylhexyl Butamido Triazone, Phenylbenzimidazol SulfonicAcid, Diethylamino Hydroxybenzoyl Hexyl Benzoate, Disodium Phenyl Dibenzimidazole Tetrasulfonate, Titandioxid, Zinkoxid und Terephthalylidene Dicamphor Sulfonic Acid.

Abhängig von dem jeweiligen Lichtschutzfilter bzw. UV-Filter liegt dessen Konzentration in dem erfindungsgemäßen kosmetischen Produkt zwischen 5 Gew.% und 30 Gew.%, insbesondere zwischen 10 Gew.% und 20 Gew.%, jeweils bezogen auf das Gewicht des anwendungsfertigen kosmetischen Produktes.

Bei einer anderen Ausgestaltung weist das erfindungsgemäße kosmetische Produkt als hautschützenden Wirkstoff Allantoin, Chlorogensäuren, Kolostrum, Lactobacillus/Algenferment, Laminaria digitata Extrakt, Laminaria japonica Extrakt, Mimosa tenuiflora Rindenextrakt, Plantago ovata Extrakt, Polygonum fagopyrum Extrakt, Kalium-Ascorbyl-Tocopherylphosphat, PVP/Eicosencopolymer, PVP/Hexadekancopolymer, Salvia officinalis Extrakt, Sophora japonica Extrakt, Sphingolipide, Spirulina platensis Extrakt, Tocopheryllinoleat, Vitis vinifera Samenextrakt und/oder Hefenbetaglucan auf, deren bevorzugte Konzentration insbesondere zwischen 0,005 Gew.% und 40 Gew.%, vorzugsweise zwischen 0,005 Gew.% und 10 Gew.%, variiert.

Zu den bevorzugten hautpflegenden Wirkstoffen, die in dem erfindungsgemäßen kosmetischen Produkt wahlweise einzeln oder als Mischung vorliegen, sind insbesondere Actelyglucosamin, Acetylglutaminsäure, Adenosin, zyklisches Adenosinphosphat, Adenosinphosphat, Adenosintriphisohat, Alchemilla vulgaris Extrakt, Ammi visnaga Extrakt, Ammoniumglycyrrhizinat, Anthemis nobilis Extrakt, Arbutin, Arctium lappa Extrakt, Asiatische Säure, Aspergillus Ferment, Atelocollagen, Avena sativa Protein, Beta-Carotin, Betaglucan, Beta-Sitosterol, Biosaccharide Gum-1, Biotin, Bombyxextrakt, Butyrospermum parkii Butter, C12-20 Isoparaffin, C14-18 Glykol, C16-36 Alkylstearat, C18-30 Glykol, C20-24 Olefin, C20-30 Glykol, C24-28 Olefin, C30-45 Alkyldimethicon, C30-50 Alkyl-Bienenwachs, C30-50 Alkylstearat, C40-60 Alkylstearat, Ceramid 1, Ceramid 1A, Ceramid 2, Ceramid 4, Ceramid 5, Ceramid 6II, Ceramid 3, DI-C12-13 Alkyltartrat, Echinacea agustifolia Extrakt, Elastin, Elastin-Aminosäuren, Folsäure, Glucuronolacton, Glutamin, Glycerylstearatcitrat, Glycin, Glycine soja Extrakt, Glycine Soja Blüte, Glycine soja Samenextrakt, Glycine Soja Öl, Glycin Soja Öl (unverseifbar), Glycin Soja Protein, Glyzyrrhetinsäure, Glycyrrhetinyl Stearat, Glycyrrhizinsäure, Guar hydroxypropylthimoniumchlorid, Harpagophytum procumbens Extrakt, Helianthus annuus Extrakt, Helianthus annuus Samenextrakt, Helianthus annuus Samenöl, Heliotropin, Hexymethyldisiloxan, Hexamidindiisethionat, Hippophae rhamnoides Öl, Hordeum vulgare Extrakt, Hydriertes Baumwollsamen Glycerid, Hydriertes Baumwollsamen Öl, hydriertes Jojobaöl, hydrierter Jojobawachs, hydrierter Lanolinalkohol, hydriertes Palm Glycerid, hydriertes Palm Glycerid Citrat, hydriertes Palmkern Glycerid, hydriertes Palm Öl, hydrierte Palm/Palmkernöl PEG- 6 Ester, hydriertes Erdnussöl, hydriertes Polyisobuten, hydriertes Rapssamenöl, hydrierte Pflanzenglyceride, hydriertes Pflanzenöl, hydrolysiertes Eiweiß, hydrolysiertes Kasein, hydrolysiertes Kollagen, hydrolysiertes Maisprotein, hydrolysierte Maisstärke, hydrolysierte DNS, hydrolysierte Eierschalenmembran (hydrolyzed egg shell membrane), hydrolysiertes Elastin, hydrolysiertes Extensin, hydrolysiertes Fibronektin, hydrolysiertes Gadidae-Protein, hydrolysierte Gelatine, hydrolysierte Glykosaminoglykane, hydrolysiertes Glycyrrhizinat, hydrolysiertes Haarkeratin, hydrolysiertes Milchprotein, hydrolysierte Seide, hydrolysiertes Soja, hydrolysiertes Sojaprotein, hydrolysierte Sojastärke, hydrolysiertes Pflanzenprotein, hydrolysiertes Weizengluten, hydrolysiertes Weizenprotein, hydrolysiertes Weizenprotein/ Dimethiconcopoliolphosphat-Copolymer, hydrolysierte Weizenstärke, hydrolysierte Hefe, hydrolysiertes Hefeprotein, hydrolysiertes Zein, Hyroxylauroylphytosphingosin, Hydroxyprolin, Hydroxypropylcyclodextrin, Hydroxypropyltrimonium hydrolyzed rice bran protein, Hydroxypropyltrimonium hydrolyzed silk, Hydroxypropyltrimonium hydrolyzed soy protein, Hydroxypropyltrimonium hydrolyzed vegetable protein, Isoamyllaurat, Isobutylmyristat, Isobutylpalmitat, Isobutylpelargonat, Isobutylstearat, Isobutyl tallowate, Isocetylbehenat, Isocetylisostearat, Isodecylneopentanoat, Isohexadecan, Isohexyllaurat, Isohexylneopentanoat, Isohexylpalmitat, Isolaurylbehenat, Isopropylisostearat, Isostearylisostearat, , Jojoba-Alkohol, Jojoba-Ester, Keratin, Keratin-Aminosäuren, Milchsäure, Lactobacillus-Ferment, Lactoferrin, Lactoglobulin, Laktose, Lactoylphytosphingosin, Lanolin, Lanolin Cera, Lanosterol, Lauramidopropylbetain, Lauryldimoniumhydroxypropyl-hydrolyisiertes Sojaprotein, Lauryldimoniumhydroxypropylhydrolisiertes Weizenprotein, Lecithin, Linolsäure, Linolensäure, Linum usitatissimum Extrakt, Linum usitatissimum Öl, Lonicera japonica Extrakt, Lysin, Lysinaspartat, Macadamia ternifolia Extrakt, Magnesiumascorbat, Magnesiumglukonat, Magnesium-PCA, Maltodextrin, Marrubium vulgare Extrakt, Medicago sativa Öl (unverseifbar), Melia azadirachta Extrakt, Methyllaurat, Methyllinoleat, Methylmyristat, Methylsilanolmannuronat, Milchaminosäuren, Moringa Samenöl, Wiesenschaumkrautsamen Öl, Moringa Butter Myristyllaktat, Myristylmyristat, Myristylneopentanoat, Oenothera biennis Extrakt, Olea europaeaExtrakt, Ophiopogon japonicus Extrakt, Oryza sativa cera, Oryza sativa Extrakt, Oryzanol, Panthenol, Pantothensäure, Papain, PCA-Dimethicon, PEA-palmitat, Phytosphingosin, Polyquaternium-47, Kaliumaspartat, Kalium-cocoyl-hydrolisiertes Kornprotein, Kaliumglycyrrhizinat, Procollagen, Prolin, Protease, PTFE, Pyridindicarbonsäure, Pyridoxal-5 Phosphat, Pyridoxin-HCI, Pyrus malus Extrakt, Quaternium-79 hydrolisiertes silk, Quaternium-79 hydrolisiertes Sojaprotein, Quaternium-79 hydrolisiertes Weizenprotein, Reseda luteola Extrakt, Resorcinolacetat, Retinol, Retinylacetat, Retinyllinoleat, Retinylpalmitat, Retinylpropionat, Ribes nigrum Extrakt, Riboflavin, Gelee Royale, Gelee Royale Extrakt, Ruscogenin, Ruscus aculeatus Extrat, Ruta graveolens Extrakt, Rutin, Saccharomyces-Ferment, Saccharomyces-Lysatextrakt, Salix alba Rindenextrakt, Salvia officinalis, Salvia officinalis Extrakt, Sericin, Serin, Serumalbumin, Serumprotein, Schellackwachs, Seidenaminosäuren, Seidenraupenlipide, Silybum marianum Extrakt, Simethicon, Simmondsia chinensis Wachs, Simmondsia chinensis Extrakt, Natriumascorbyl/Cholesteryl-Phosphat, Natriumhyaluronat-Kreuzpolymer, Natriumhyaluronatdimethylsilanol, Natriumisethionat, Natriumlauroylisethionat, Natriumlauroyl Haferaminosäuren, Natriumlauroylsarkosinat, Natriumlauroyl Seideaminosäuren, Natriumlevulinat, Natrium-PCA, Natrium-PCA Methylsilanol, Natriumpolyglutamat, Sorbitol, Sojasterol, Sphingolipide, Squalan, Stearamidopropylbetain, Stearylglycyrrhetinat, Stearyllaktat, Saccharose, Sonnenblumen-Samenöl-Glyceride, Symphytum officinale Blattextrakt, Theobroma cacao Extrakt, Tocopheryllinoleat, Triticum vulgare Extrakt, Troxerutin, Tyrosin, Ubichonon, Undecylalkohol, Urea, pflanzliche Glyceridphosphate, Verbena officinalis Extrakt, Vitamine und deren Derivate, vorzugsweise Vitamin A und E, Weizenaminosäuren, Zea mays, Zinkaspartat, Zink-DNA, Zinkglukoheptonat, Zinkglukonat, Zinkglutamat, Zink hydrolisiertes Collagen und/oder Zink-PCA zu nennen.

Abhängig von dem jeweiligen Wirkstoff und dem beabsichtigten Verwendungszweck variiert die bevorzugte Konzentration der zuvor aufgeführten hautpflegenden Wirkstoffen zwischen 0,005 Gew-⁰/o und 40 Gew.%.

Bei den glättenden oder faltenvermindernden Wirkstoffe sind insbesondere in dem erfindungsgemäßen kosmetischen Produkt Boswelia serrata Extrakt, Centella asiatica Extrakt, Glycyrrhiza glabra, Glycyrrhiza glabra Extrakt, Morus alba Extrakt, Niacin, Niacinamid, Persea gratissima Extrakt, Sericin, Pentapeptide, Hexapeptide, insbesondere Hexa-Peptide-2und/oder Hexapeptide-9, Heptapeptide, Kupfer-Peptide, Wachstumsfaktoren der TGF Beta Familie, MPC Milch Peptide, MTP Milch Tripeptide, Palmitoyloligopeptide/Matrikine, insbesondere Pal-KTTKS (Hersteller: Sederma) und/oder Pal-VGVAPG (Hersteller: Sederma), Acetylhexapeptide 3, Palmitoylpentapeptide, Palmitoyltripeptide-5, Serilesine (= Laminin, Hersteller: Lipotec), Lipeptide (=Oligopeptide, Hersteller: Lipotec), Tripeptide 10-citrullin, Aldenine (Hersteller: Lipotec), Polygamma-Glutamine-Säure, Alanin, Serin, Glycin, Arginin, Glutaminsäure, Histidin und Spirulina maxima Extrakt in einer bevorzugten Konzentration zwischen 0,001Gew.% und 40 Gew.% enthalten.

Zur Gruppe der geschmeidigmachenden Wirkstoffe gehören insbesondere Arachis Hypogaea Öl, Arctium Lappa Samenöl, Avena Sativa Extrakt, Behenylalkohol, Borago Officinalis Extrakt, Borago Offincinalis Samenöl, Brassica Campestris Oleifera Öl, Butyrospermum Parkii Butter, Butyrospermum Parkii Butter-Extrakt, Butyrospermum Parkii Butter (unverseifbar), Calendula Officinalis Extrakt, Calendula Officinalis Öl, Candelilla Cera, Canola-ÖI, Caprylylglycol, Carthamus Tinctorius Extrakt, Carthamus Tinctorius Öl, Cera Alba, Cera Carnauba, Ceratonia Siliqua Extrakt, Cetearylalkohol, Cetearyl-Isononanoat, Cetylalkohol, Cetyl-Ethylhexanoat, Cetylpalmitat, Cetylstearat, Cholesterin, Cholesteryl-Hydroxystearat, Cholesteryl Isostearat, Cholesteryl Macadamiat, Cholesteryl Nonanoat, Cholesteryl Stearat, Cocoglyceride, Cocos Nucifera Extrakt, Cocos Nucifera Öl, Cocoyl-Glutaminsäure, Coenzym A, Coriandrum Sativum Samenöl, Cyclohexasiloxan, Cyclomethicon, Cyclopentasiloxan, Cyclotetrasiloxan, Cyclotrisiloxan, Dibutyladipat, Dibutylsebacat, Diethyl-Sebacat, Diethyl-Succinat, Dihydrocholesterol, Dihydrocholesteryl-Butyrat, Dihydrocholesteryl-Isostearat, Dihydrocholesteryl-Macadamiat, Dihydrocholesteryl-Nonanoat, Dihydrocholesteryl-Octyldecanoat, Dihydrocholesteryl-Oleat, Diisobutyl-Adipat, Equisetum Arvense Extrakt, Erucyl-Arachidat, Erucyl-Erucat, Ethyl-Olivat, Glyceryl-Caprat, Glyceryl-Caprylat, Glyceryl-Caprylat/Caprat, Glyceryl-Cocoat, Glyceryl-Diarachidat, Glyceryl-Dibehenat, Glyceryl-Dierucat, Glyceryl-Dihydroxystearat, Glyceryl-Diisopalmitat, Glyceryl-Diisostearat, Glyceryl-Dilaurat, Glyceryl-Dilinoleat, Glyceryl-Dimyristat, Glyceryl-Dioleat, Glyceryl-Dipalmitat, Glyceryl-Dipalmitoleat, Glyceryl-Diricinoleat, Glyceryl-Distearat, Glyceryl-Erucat, Glyceryl-Ethylhexanoat/Stearat/Adipat, hydriertes Glyceryl-Sojat, Glyceryl-Hydroxystearat, Glyceryl-Isostearat, Glyceryl- Isostearat/Myristat, Glyceryl-Isostearate, Glyceryl-Lanolat, Glyceryl-Laurat, Glyceryl-Laurat/Oleat, Glyceryl-Linoleat, Glyceryl-Myristat, Glyceryl-Oleat, Glyceryl-Oleat/Elaidat, Glyceryl-Palmitat, Glyceryl-Palmitat-Laktat, Glyceryl-Palmitat/Stearat, Glyceryl-Ricinoleat, Glyceryl-Ricinoleat-SE, Glyceryl- Sesquioleat, Glyceryl-Stearat, Glyceryl-Stearat-Citrat, Glyceryl-Stearat-Diacetat, Glyceryl-Stearat-Laktat, Glyceryl-Stearat-Succinat, Glyceryl-Stearat/ Acetat, Glyceryl-Stearat/Maleat, Glyceryl-Tallowat, Glyceryl-Triacetyl-Hydroxystearat, Glyceryl-Triacetyl-Ricinoleat, Glyceryl/Sorbitol-Oleat/ Hydroxystearat, Glykosphingolipide, Glycyrrhiza Glabra, Glycirrhiza Glabra Extrakt, Baumwollöl, Helianthus Annuus Extrakt, Helianthus Annuus Samenöl, Hexamidin-Diisethionat, Hippophae Rhamnoides Öl, Hordeum Vulgare Extrakt, Humulus Lupulus Extrakt, hydrierte Coco-Glyceride, hydriertes Jojoba-Öl, hydriertes Jojobawachs, hydriertes Lanolin, hydrierte Palmglyceride, hydriertes Palmkernöl, hydriertes Polydecen, hydriertes Rapsöl, hydriertes Pflanzenöl, hydrolisiertes Collagen, Hypericum Perforatum Öl, Isohexadecan, Isopropyl-Isostearat, Lauryldimonium-Hydroxypropyl-Collagen Limnanthes Alba Samenöl, Macadamia Ternifolia Extrakt, Palmitoylcollagen (hydrolysiert), Panax Ginseng Extrakt, Paraffin, Paraffinum Liquidum, Rices Nigrum Extrakt, Ribes Nigrum Öl, Simmondsia Chinensis Cera, Simmondsia Chinensis Extrakt, Simmondsia Chinensis Öl, Solanum Lycopersicum Öl, Sorbitandistearat, Candelilla Wachs (synthetisch), Carnauba(synthetisch), Japanwachs (synthetisch), Jojobaöl (synthetisch), Wachs (synthetisch), Triethylhexyl-Citrat und Verbena Officinalis Extrakt. Die bevorzugte Konzentration dieser geschmeidigmachenden Wirkstoffe liegt zwischen 0,5 Gew.% und 40 Gew.%.

Zu der Gruppe der hautaufhellenden Wirkstoffe, die in dem erfindungsgemäßen kosmetischen Produkt vorhanden sein können, gehören insbesondere Magnesium Ascorbylphosphat, Di-Natriumascorbylphosphat, Tetrahydrodiferuloyl-methan, Lepidium Sativum Sprossen Extrakt, Hydroquinon, Tretinoin, Azelain Säure, Koji Säure, Kojic Dipalmitat, Mequinol, Arbutin, Bayberry Extrakt, Paper Mulberry Extrakt, Glabridin, Licorice Extrakt, Ascorbinsäure, Glyxyrrhiza Uralensis Extrakt, Melanostat, Octadecendioc Acid, Phenylpropanoide, Zinc-Glycin Komplexe, Tretionin, Waltheria Indica Blatt Extrakt, Hydroxyphenoxy Propionic Acid, Undecylenoyl Phenylalanin, Ascorbyl Tetraisopalmitat, Mandresey Extrakt, Ascorbinsäure 2-Glucosid und 4-(1-Phenylethyl)1,3-Benzenediol. Abhängig von dem jeweiligen Wirkstoff variiert dessen Konzentration bevorzugte zwischen 0,01 Gew.% und 7 Gew.%.

Als bräunende Wirkstoffe sind bevorzugt Acetyltyrosin, Erythrulose und Dihydroxyaceton in einer bevorzugten Konzentration zwischen 0,1 Gew.% und 10 Gew.% in dem erfindungsgemäßen kosmetischen Produkt enthalten.

Bei den desodorierenden Wirkstoffen werden in dem erfindungsgemäßen kosmetischen Produkt insbesondere Aluminiumchlorhydrat/, Triethyl Citrate, Silver Citrate, Sodium Caproyl/Lauroyl Lactylate in einer bevorzugten Konzentration zwischen 0,1 Gew.% und 15 Gew.% zu nennen.

Zur Gruppe der enthaarenden Wirkstoffe gehören insbesondere Ammonium-Thiolaktat, Calcium-Thioglycolat, Mercaptopropionsäure, Kalium-Thioglycolat, Natrium-Thioglycolat und Mercaptopropionsäure in einer bevorzugten Konzentration zwischen 5 Gew.% und 10 Gew.%.

Feuchthaltende Wirkstoffe sind vorzugsweise ArgininPCA, Butylenglycol, Butyloctanol, Calciumglukonat, Carboxymethyl-Chitosan-Succinamid, Chitosa PCA, Copper-Acetyl-Tyrosinat-Methylsilanol, Copper PCA, Copper-PCA-Methylsilanol, Serin, Glycin, Alanin, Sodium Polyaspertat, Betain, Urea, Dikalium-Glycyrrhizat, Erythritol, Ethoxydiglycol, Ethylhexyl-PCA, Galactonolakton, Glucamin, Gluconsäure, Gluconolakton, Glukuronsäure, Glutaminsäure, Glycyrrhizinsäure, Hyaluronsäure, Inositol-Hexa-PCA, Isomalt, Lysin PCA, Magnesium PCA, Maltitol, Phytantriol, Kalium PCA, Saccharid-Hydrolysat, Natriumcarboxymethyl-Chitin, Natrium-Hyaluronat, Natrium-Hyaluronat- Kreuzpolymer, Natrium-Hyaluronat-Dimethylsilanol, Natriumlaktat, Natrium PCA, Natrium-Polyaspartat, Natrium-Polyglutamat, Sorbitol, Sojasterol, Urea, Xylitol, Xylose, Fillagrin stimulierende Substanzen, wie Madecassoside, Weizenproteine, Alteromonas Ferment Filtrate , Hydrolyzed Beta-Glucan (Hydreis, Impag), Revidrate (Sederma), PCA (Pyrrolidoncarbonsäure) und Derivate in einer bevorzugten Konzentration zwischen 0,05 Gew.% und 25 Gew.%, bezogen auf das Gewicht des anwendungsfertigen kosmetischen Produktes.

Für die Pflege bzw. Behandlung überempfindlicher Haut sind insbesondere die Wirkstoffe Butylcyclohexanol, vorzugsweise Butylcyclohexanole und insbesondre 4-t-Butylcyclohexanol und Palmitoyl tripeptide-8 zu nennen, die in einer bevorzugten Konzentration zwischen 0,01 Gew.% und 2,5 Gew.% in dem erfindungsgemäßen kosmetischen Produkt vorliegen.

Eine besonders hohe kosmetische Wirksamkeit in bezug auf die Erhaltung einer gesunden Hautbarriere und in Bezug auf die Wiederherstellung einer geschädigten Hautbarriere wird bei einer Weiterbildung des erfindungsgemäßen kosmetischen Produktes dadurch erreicht, daß das erfindungsgemäße Produkt eine Mischung aus Butylcyclohexanol, insbesondere aus tertiären Butylcyclohexanolen und vorzugsweise aus 4-t-Butylcyclohexanol mit Propylenglycol und/oder Pentylenglycol aufweist.

Um mit dem erfindungsgemäßen kosmetisches Produkt insbesondere infizierte, gereizte oder erkrankte Hautbereiche, zu behandeln, sieht eine Weiterbildung vor, daß hierbei das erfindungsgemäße kosmetische Produkt einen anti-entzuhdlichen Wirkstoff enthält, der ausgewählt ist aus der Gruppe, die Ursolsäure, Soja-Sterol, 18-beta-Glycyrrhetinsäure, Gamma-Oryzanol, Ferula-Säure, Avenanthramide, Boswellinsäure, Asiaticoside, Madecassoside, CM Glucan, Troxerutin, Rutin, Monoalkanolamide, Rosmarinsäure, Ringelblumenextrakt, Johanniskrautextrakt, Cardiospermum Halicacabum Extrakt, Kamillenextrat, Sonnenhutextrakt und Derivate der zuvor genannten antientzündlichen bzw. juckreizlindernden Wirkstoffe umfaßt, wobei die bevorzugte Konzentration zwischen 0,01 Gew.% und 5 Gew.% liegt.

Um bei dem erfindungsgemäßen kosmetischen Produkt eine Prophylaxe gegen Insektenstiche zu erreichen, weist diese Ausgestaltung des erfindungsgemäßen Produktes einen Wirkstoff auf, der aus der Gruppe ausgewählt ist, die Icaridin, Nelkenöl, Citronellal, Zedernholzöl, Lavelöl, Zimtöl, Permethrin und Crotamiton umfaßt. Hierbei verhindern insbesondere diese Wirkstoffe, daß der Anwender insbesondere gegen Stiche von Mücken, Flöhen, Läusen und/oder Zecken geschützt ist, wobei die bevorzugte Konzentration zwischen 0,1 Gew.% und 10 Gew.% variiert.

Bevorzugt enthält das erfindungsgemäße kosmetische Produkt als rückfettenden Wirkstoff Weizenkeimglyceride in einer bevorzugten Konzentration zwischen 0,1 Gew.% und 5 Gew.%.

Als feuchtigkeitsspendende Wirkstoffe sind insbesondere Dimethylsilanol-Hyaluronat, Glycin-Soja-Extrakt, Glycyrrhiza Glabra und/oder Mangan PCA in dem erfindungsgemäßen kosmetischen Produkt in einer bevorzugten Konzentration zwischen 0,05 Gew.% und 5 Gew.% enthalten.

Eine weitere Ausgestaltung des erfindungsgemäßen kosmetischen Produktes, das insbesondere für die Pflege und Behandlung von gegenüber Noxe überempfindliche Haut sowie gegen neurogen indizierten Hautentzündungen verwendet wird, weist als Wirkstoffe Butylcyclohexanol, Resolvin, Farnesyl Pyrophosphate, Capsazepine, Cinnamide, Carboxamide und/oder Palmitoyl tripeptide-8 auf, wobei die bevorzugte Konzentration dieser Wirkstoffe zwischen 0,01 Gew.% und 1 Gew.% liegt.

Die vorliegende Erfindung betrifft desweiteren ein Konzentrat zur Herstellung des zuvor beschriebenen erfindungsgemäßen kosmetischen Produktes, wobei das Konzentrat durch Verdünnung mit einem wäßrigen System herstellbar ist, und das Konzentrat mindestens ein hydriertes Phospholipid, Wasser, mindestens einen zwei- und/oder dreiwertigen Alkohol und mindestens ein pflanzliches Wachs enthält. Erfindungsgemäß wird vorgeschlagen, daß das Konzentrat unter Ausbildung des kosmetischen Produktes in einem Volumenverhältnis zwischen 1:0,3 bis 1:15 mit dem wäßrigen System verdünnbar ist, wobei das Konzentrat eine solche Konzentration an hydriertem Phospholipid aufweist, daß abhängig von der erwünschten Verdünnung das hieraus durch Verdünnung hergestellte kosmetische Produkt mindestens 0,7 Gew.% des hydrierten Phospholipids enthält. Desweiteren variiert im erfindungsgemäßen Konzentrat das Gewichtsverhältnis von hydriertem Phospholipid zu dem pflanzlichen Wachs zwischen 1:0,3 und 1:1,5, insbesondere zwischen 1:0,7 und 1:1,1. Im erfindungsgemäßen Konzentrat liegt ferner das hydrierte Phospholipid zumindest teilweise in einer ortho rhombischen lamellaren kristallinen Struktur vor, wobei in dem erfindungsgemäßen Konzentrat das Wachs in der ortho rhombischen lamellaren kristallinen Struktur eingelagert und/oder an der ortho rhombischen lamellaren kristallinen Struktur angelagert ist.

Für das erfindungsgemäße Konzentrat gelten alle die zuvor für das erfindungsgemäße kosmetische Produkt beschriebenen Vorteile analog oder identisch, was ebenfalls für die vorstehend beim erfindungsgemäßen kosmetischen Produkt beschriebenen Ausführungsformen und Ausgestaltungen gilt. Ein besonderer Vorteil des erfindungsgemäßen Konzentrates liegt darin begründet, daß aus einem einzigen Konzentrat eine Vielzahl von unterschiedlich zusammengesetzten kosmetischen Produkten herstellbar ist und daß desweiteren die Handhabung und der Transport des Konzentrates im Vergleich zu einer Vielzahl in kleinen Einheiten abgefüllten kosmetischen Produkten wirtschaftlich besonders günstig ist und darüber hinaus auch noch vereinfacht wird.

Eine weitere Ausgestaltung des erfindungsgemäßen Konzentrates schlägt vor, daß hierbei das wäßrige System mindestens einen kosmetischen Wirkstoff aufweist. Diese Ausgestaltung des erfindungsgemäßen Konzentrates weist zu dem bereits vorstehend beschriebenen Vorteilen noch zusätzlich den Vorteil auf, daß bei besonders gegenüber abbauempfindlichen Wirkstoffen diese Wirkstoffe unmittelbar vor der Herstellung dem wäßrigen System zugeführt und mit dem Konzentrat vermischt werden, so daß für derartige, mit besonders empfindlichen kosmetischen Wirkstoffen zu versehene kosmetische Produkte diese kosmetischen Produkte erst unmittelbar vor dem Versand hergestellt werden.

Die vorliegende Erfindung betrifft desweiteren die Verwendung des zuvor beschriebenen erfindungsgemäßen kosmetischen Produktes sowie des ebenfalls zuvor dargelegten erfindungsgemäßen Konzentrates.

Insbesondere wird das erfindungsgemäße Produkt oder das erfindungsgemäße Konzentrat zur adjuvanten Pflege, zur Vorbeugung und/oder zur Behandlung bei infizierter, gereizter oder erkrankter Haut, insbesondere bei Psoriasis, Endogenem Ekzem, Strahlenschäden, Lichtdermatose, Perlèche, Aktinische Keratose, Kontaktdermatitis, seborrhoische Dermatitis, Windeldermatitis, Couperose, Dekubitus, Ichthyosis, Herpes labialis, Lentigo, Perioorale Dermatitis, Skabies, Urtikaria, Verbrennungen ersten Grades und/oder von solchen Hautstörungen eingesetzt, die durch ionisierende Strahlung und/oder durch Zytostatika erzeugt werden.

Abhängig von der zuvor angesprochenen Verwendung werden hierfür in dem erfindungsgemäßen Produkt oder dem erfindungsgemäßen Konzentrat die Wirkstoffe eingesetzt, die vorstehend im Patentanspruch 17 genannt und in der zugehörigen Beschreibung als bevorzugte Wirkstoffe hervorgehoben sind, so daß zur Vermeidung von Wiederholungen hierauf verwiesen wird.

Um die zuvor wiederholt beschriebene ortho rhombische lamellare kristalline Struktur in dem erfindungsgemäßen kosmetischen Produkt und dem erfindungsgemäßen Konzentrat nachzuweisen, wird eine Röntgenstrukturanalyse durchgeführt, wie sie nachstehend bei den Ausführungsbeispielen noch im Detail beschrieben ist.

Abhängig von dem jeweiligen Verhältnis von hydriertem Phospholipid zu Wachs variiert die Phasenübergangstemperatur des zumindest teilweise in einer ortho rhombisch lamellaren kristallinen Struktur vorliegenden hydrierten Phospholipids, an das das Wachs eingelagert und/oder angelagert ist, zwischen 72 °C und 95 °C.

Der in dieser Beschreibung verwendete Begriff und/oder ist sowohl additiv als auch alternativ zu verstehen. Ebenso deckt eine im Singular verwendete Formulierung auch den Plural ab und eine im Plural verwendete Formulierung den Singular.

Reiskleiewachs wird auch vielfach als rice bran wax oder als Reiskeimwachs bezeichnet.

Vorteilhafte Weiterbildungen des erfindungsgemäßen kosmetischen Produktes und des erfindungsgemäßen Konzentrates sind in den Unteransprüchen angegeben.

Das erfindungsgemäße kosmetische Produkt wird in Verbindung mit den schematischen Zeichnungen gemäß der Figuren 1 bis 3, 4, 5, 5A und 6 der beiden Wirksamkeitsstudien, und den Ausführungsbeispielen 1 bis 12 näher erläutert. Es zeigen:
- Figur 1: eine schematische Abbildung einer ortho rhombischen lamellaren kristallinen Struktur;
- Figur 2: eine schematische Abbildung einer einzigen Ebene der in Figur 1 abgebildeten ortho rhombischen lamellaren kristallinen Struktur;
- Figur 3: eine schematische Abbildung einer einzigen Ebene einer hexagonalen lamellaren kristallinen Struktur;
- Figur 4, 5 und 5A: die Röntgenstrukturanalysenergebnisse; und
- Figur 6: das Ergebnis einer ersten Wirksamkeitsstudie.

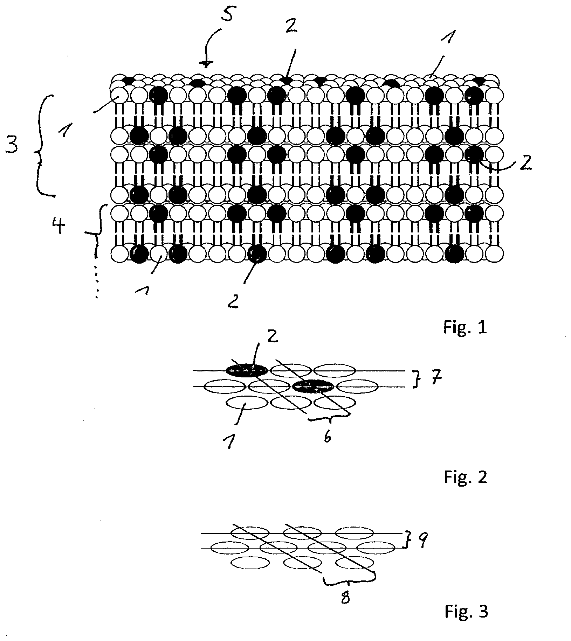

Die Figur 1 bildet schematisch eine ortho rhombische lamellare kristalline Struktur ab, die das hydrierte Phospholipid und insbesondere das hydrierte Phosphatidylcholin unter den Herstellungsbedingungen ausbildet, wie sie bei den Ausführungsbeispielen 1 bis 12 im Detail beschrieben sind. Die hydrierten Phospholipidmoleküle (beispielhaft mit 1 bezeichnet) sind durch einen weißen Kreis gekennzeichnet. An bzw. in diese Struktur sind schwarz abgebildete Wachsmoleküle (beispielhaft mit 2 bezeichnet) angelagert und/oder eingelagert.

Die abgebildete Struktur gemäß Figur 1 weist beispielhaft eine erste planare Doppelmembranschicht 3 und eine nur teilweise gezeigte planare zweite Doppelmembranschicht 4 auf, wobei diese beiden Doppelmembranschichten 3 und 4 sandwichartig übereinander angeordnet sind.

Die vollständig abgebildete Doppelmembranschicht 3 besteht aus zwei Lagen, wobei innerhalb der Lagen die einzelnen Moleküle des hydrierten Phospholipids 1 und des Wachses 2 so ausgerichtet sind, wie dies in der Figur 1 abgebildet ist.

In Figur 2 ist die ortho rhombische lamellare kristalline Struktur schematisch an einem Ausschnitt in einer einzigen Ebene, die der Ebene der in Figur 1 mit 5 gekennzeichnete Ebene entspricht und somit eine Draufsicht auf die in Figur 1 gezeigte lamellare Struktur darstellt, abgebildet.

In Richtung dieser Ebene 5 gesehen beträgt bei der in Figur 2 dargestellten ortho rhombischen lamellaren kristallinen Struktur der mit 6 bezeichnete Molekülabstand 3,71 Å, während der seitliche mit 7 bezeichnete Abstand der Moleküle 4,16 Å beträgt.

Im Gegensatz zur Figur 2 zeigt die Figur 3 eine hexagonale lamellare kristalline Struktur. Hierbei betragen die Molekülabstände 8 und 9, die den Abständen 6 und 7 der in der Figur 2 gezeigten Struktur bezüglich ihrer Lage entsprechen, jeweils 4,16 Å.

Die zuvor aufgezeigten Unterschiede in den Molekülabständen, einerseits von 3,71 Ä und 4,16 Å bei der ortho rhombischen lamellaren kristallinen Struktur gemäß Figur 2 und andererseits von 4,16 Å (in beiden Richtungen) bei der hexagonalen lamellaren kristallinen Struktur gemäß Figur 3, lassen sich durch die nachfolgend beschriebene Röntgenstrukturanalyse eindeutig nachweisen.

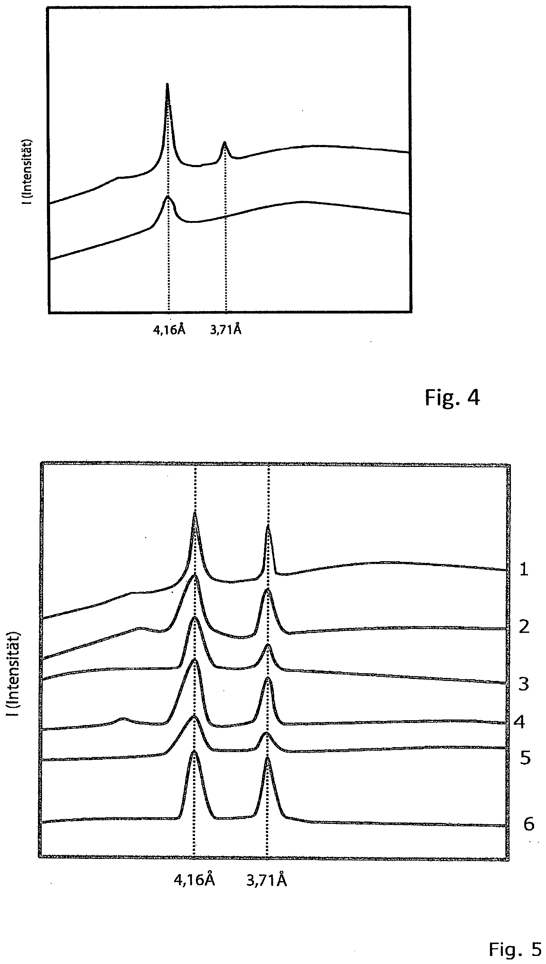

Dieser Nachweis wird durch die Figur 4 belegt, die das entsprechende Röntgenstrukturanalysenergebnis für die bei der nachfolgend beschriebenen ersten vergleichenden Wirksamkeitsstudie eingesetzten beiden Formulierungen, dort bezeichnet mit Formulierung A (erfindungsgemäß) und Vergleichsrezeptur B, wiederspiegelt.

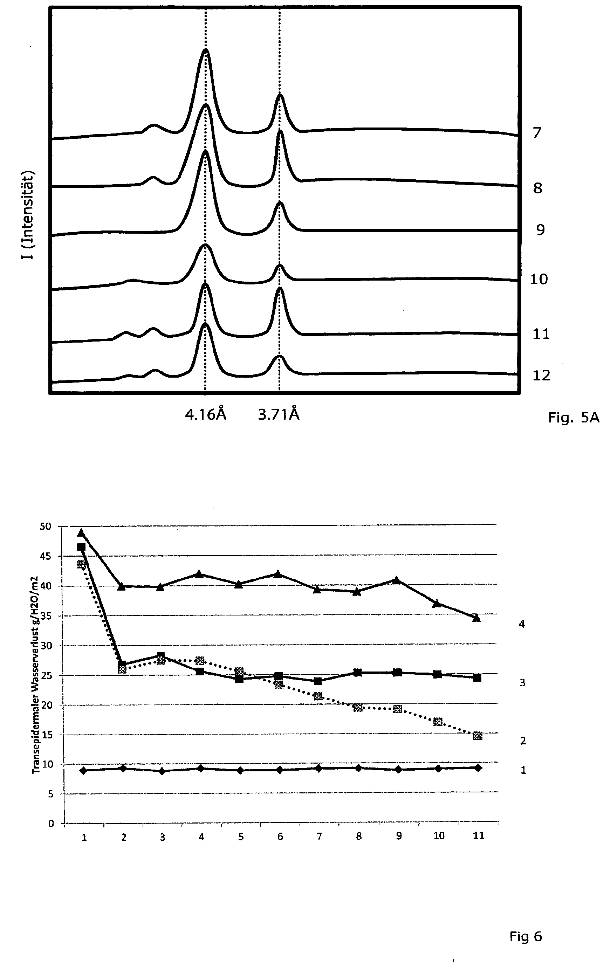

Ebenso belegen die Figur 5 und 5A, daß alle in den Ausführungsbeispielen 1 bis 12 beschriebenen Zusammensetzungen eine ortho rhombische lamellare kristalline Struktur ausbilden, wobei am rechten Rand dieser beiden Abbildungen durch die Bezugszeichen 1 bis 6 und 7 bis 12 die Ausführungsbeispiele 1 bis 6 und 7 bis 12 identifiziert sind. Alle dort abgebildeten Röntgenstrukturanalysenergebnisse weisen zwei Peaks auf, einen bei 3,71 Ä und einen zweiten Peak bei 4,16 Å.

Zu den Figuren 4, 5 und 5A ist anzumerken, daß die Intensität nicht kalibriert ist, da es sich bei der Figur 4 um die Wiedergabe der zwei Röntgenstrukturanalysenergebnisse der beiden in der ersten Wirksamkeitsstudie miteinander verglichenen Formulierungen A und B und bei Figur 5 um die Wiedergabe der sechs Röntgenstrukturanalysenergebnisse zu den Ausführungsbeispielen 1 bis 6 handelt, die jeweils in einem Diagramm zusammengefaßt sind. Die Figur 5A gibt die Ergebnisse der Röntgenstrukturanalyse der Ausführungsbeispiele 7 bis 12 graphisch wieder.

### Nachweis der ortho rhombischen Struktur

Die in der vorliegenden Anmeldung aufgeführten ortho rhombischen lamellaren kristallinen Strukturen und die hexagonale lamellare kristalline Struktur werden wie folgt durch Röntgenstrukturanalyse nachgewiesen:
Apparatur: MARCCD-345X Röntgenbeugungsapparatur
   - rotierende Cu-Anode Röntgengenerator, λ=1.5418Å
   - Strahl : Durchmesser: 1x1mm²
   - Transmissionsgeometrie: Strahl perpendikular zur Probenfläche
   - Detektion: Bildflächenkamera, 2300x2300 Pixels, Pixelgröße: 150x150µm²
   - Proben-Detektorabstand: 250mm
   - Bereich der Retikularabstände: 50Å bis 2,7Å, Vorwärtstreuungsvektor S (=1/d):0,020 bis 0,37Å⁻¹
   - Zeitliche Exposition: 900 s.
   - T=21°C, 40 % relative Raumfeuchtigkeit
Datenanalyse:
   - Subtraktion des Hintergrundsignals (Luft) gemessen außerhalb der Probe
   - Software: FIT2D

Die untersuchten Proben, bei denen im Röntgendiagramm (Figur 4) ein einziger Peak bei 4,16 Å auftritt, ist charakteristisch für hexagonale kristalline lamellare Strukturen, während solche Proben, bei denen das Röntgendiagramm einen ersten Peak bei 4,16 Å und einen zweiten Peak bei 3,71 Å (Figuren 4, 5 und 5A) zeigt, sind charakteristisch für ortho rhombische lamellare kristalline Strukturen. Dementsprechend weist die Formulierung A (erfindungsgemäß) eine ortho rhombische lamellare kristalline Struktur und die Vergleichsrezeptur B eine hexagonale kristalline Struktur auf.

### Erste vergleichende Wirksamkeitsstudie

Es wurde eine erste vergleichende Wirksamkeitsstudie zwischen einer Ausführungsform des erfindungsgemäßen kosmetischen Produktes mit ortho rhombischen lamellaren kristallinen Struktur und einer Formulierung mit hexagonaler lamellarer Struktur (Vergleichsformulierung) durchgeführt.

Das bei diesem Vergleich verwendete hydrierte Phospholipid enthält wie bei den nachfolgend beschriebenen Ausführungsbeispielen 1 bis 12 eine Konzentration an hydriertem Phosphatidylcholin von 93 Gew.% ± 3 Gew.%, bezogen auf das Gewicht des hydrierten Phospholipids.

Die Zusammensetzung der erfindungsgemäßen Formulierung A sowie der Vergleichsformulierung B war wie folgt:

| Phase | Inhaltsstoffe | erfindungsgemäße Formulierung A in Gew.% | Vergleichsrezeptur B in Gew.% |
|---|---|---|---|
| 1 | hydriertes Phospholipid | 1,50 | 1,50 |
| 1 | Isostearyl Isostearate | 15,00 | 15,00 |
| 1 | Reiskleiewachs | 1,20 | 0,10 |
| 1 | Glycerin 99,5 % | 3,00 | 3,00 |
| 1 | Pentylene Glycol | 5,00 | 5,00 |
| 2 | Wasser | 74,30 | 75,40 |
| | Σ | 100,00 | 100,00 |

Die für die erste vergleichende Wirksamkeitsstudie erforderlichen zwei Formulierungen wurden nach demselben Herstellungsverfahren hergestellt.

Phase 1 wurde unter gleichmäßigem Rühren auf 90 °C erwärmt. Anschließend wurde Phase 2 ebenfalls auf 90 °C erwärmt. Bei 90 °C wurde Phase 2 zu der Phase 1 gegeben und anschließend bei 15.000 U/min mittels Ultra Turrax 3 Minuten homogenisiert. Anschließend wurde die Mischung auf 77 °C unter Homogenisation (12.000 U/min mittels Ultra Turrax) mit einer Abkühlrate von 1,5 °C/min abgekühlt.

Die entstandene Prädispersion wurde mittels Hochdruckhomogenisator feinstdispergiert. Es waren 3 Zyklen bei 800 bar erforderlich. Anschließend wurde das Gemisch unter Rühren auf 25 °C (Abkühlrate 1 °C/Min) abgekühlt.

Die so erstellten beiden Formulierungen wurden der nachfolgend beschriebenen Inprozesskontrolle unterzogen. Beide Formulierungen waren eine homogene Dispersion mit gleichmäßig großen Teilchen.

Die vergleichende Wirksamkeitsstudie wurde mit 10 Probanden durchgeführt. Bei den Probanden handelte es sich um Frauen im Alter zwischen 37 und 52 Jahren. Das Durchschnittsalter betrug 45 Jahre. Es lagen keine testrelevanten Erkrankungen oder Hautveränderungen bei den Probanden vor. Als Wirksamkeitskriterium diente der transepidermale Wasserverlust, der mit Hilfe des Aquaflux AF 200-Meßgerätes (Hersteller: Firma Biox) gemessen und ausgewertet wurde. Die Auswertung und Dokumentation erfolgte mittels des von der Firma BIOX bereitgestellten 64 Bit Softwaresystems.

Am ersten Tag der Studie wurden bei jedem Probanden im Abstand von 5 cm von der Ellenbogenbeuge des rechten und linken Unterarmes auf einer Fläche von 2 x 2 cm vier Hautareale gekennzeichnet. An drei der vier markierten Hautareale wurden bewußt Barrierestörungen durch eine Reizung des jeweiligen Hautareals mit 0,0125 ml einer 15 %iger Natronlauge erzeugt. Die Haut zeigte vor der Applikation der Natronlauge keine Reizungen oder Verletzungen. Die Natronlauge wurde mit einem Plastikspatel auf die jeweiligen Hautareale verteilt. Nach fünf Minuten Einwirkzeit wurde die Natronlauge durch Spülen mit isotoner Natriumchloridlösung entfernt, hiernach wurde das gereizte Hautareal abgetupft und 20 Minuten an der Luft trocknen gelassen. Diese so erzeugte Hautbarrierestörung wurde anschließend an die 20 minütige Trocknung nochmals identisch wiederholt, jedoch nur für eine Einwirkzeit der Natronlauge von 3 Minuten. Die so behandelte Haut wurde wie vorstehend beschrieben gespült, abgetupft und getrocknet.

Nach dieser Reizung zeigte die Haut eine geringfügige Quellung und Rötung im Sinne eines Erythems. Tiefer gehende Hautschäden, welche über den Bereich der Epidermis hinausgehen, konnten nicht festgestellt werden. Der transepidermale Wasserverlust wurde von allen vier Hautarealen (3 geschädigte und Vergleichsfläche) bestimmt und ist als "Ausgangswert" in der nachfolgenden Tabelle ausgewiesen. Der transepidermale Wasserverlust nach Schädigung wies einen Mittelwert von 45,07g H₂O/m² auf. Die Standardabweichung betrug 4,2g H₂O/m².

20 Minuten nach der zuvor beschriebenen zweiten Schädigung wurden jeweils auf ein geschädigtes Hautareal an jedem Arm die erfindungsgemäße Formulierung A (0,01 ml, rechter Unterarm) und die Vergleichsrezeptur B (0,01 ml, linker Unterarm) unter Verwendung einer Mikroliter Spritze auf die gereizten Hautareale aufgetragen und mit einem Spatel verteilt.

Die anderen markierten Hautflächen blieben ohne Behandlung.

Am ersten Tag der Schädigung wurden 9 Messungen des transepidermalen Wasserverlustes in einem zeitlichen Abstand von jeweils 20 Minuten durchgeführt.

Am Morgen des zweiten und am Morgen des dritten Tages wurden erneut die zu vergleichenden Formulierungen auf die entsprechenden Hautareale in der zuvor angegebenen Menge und Weise aufgetragen.

Am zweiten und dritten Tag nach etwa fünf Stunden nach dem weiteren Auftragen der zu vergleichenden Formulierungen wurden nochmals die transepidermalen Wasserverluste gemessen. Alle Ergebnisse der Messungen sind der nachfolgenden Tabelle wiedergegeben.

**Tabelle 1**

| Zeit | Transepidermaler H₂O Verlust keine Schädigung | Durchschnittswert transepidermaler H₂O Verlust erfindergemäße Formulierung | Durchschnittswert transepidermaler H₂O Verlust ohne Behandlung | Durchschnittswert transepidermaler H₂O Verlust Vergleichsformulierung |
|---|---|---|---|---|
| | | rechter Unterarm | | linker Unterarm |
| erster Tag | | | | |
| Ausgangswert nach Schädigung | 8,94 | 43,61 | 48,95 | 46,53 |
| 20 min nach Applikation | 9,31 | 25,99 | 39,93 | 26,77 |
| 40 min nach Applikation | 8,78 | 27,46 | 39,83 | 28,25 |
| 60 min nach Applikation | 9,21 | 27,4 | 41,94 | 25,6 |
| 80 min nach Applikation | 8,85 | 25,57 | 40,18 | 24,27 |
| 100 min nach Applikation | 8,91 | 23,34 | 41,9 | 24,75 |
| 120 min nach Applikation | 9,12 | 21,32 | 39,27 | 23,89 |
| 140 min nach Applikation | 9,18 | 19,41 | 38,89 | 25,27 |
| 160 min nach Applikation | 8,87 | 19,09 | 40,79 | 25,25 |

| zweiter Tag (zweite Applikation der zu vergleichenden Formulierungen) | | | | |
|---|---|---|---|---|
| 24 Stunden nach Schädigung | 9,01 | 16,91 | 36,89 | 24,92 |

| dritter Tag (dritte Applikation der zu vergleichenden Formulierungen) | | | | |
|---|---|---|---|---|
| 48 Stunden nach Schädigung | 9,12 | 14,53 | 34,38 | 24,35 |

Die vorstehende Tabelle 1 gibt die Messung des transepidermalen Wasserverlustes bei der ersten vergleichenden Wirksamkeitsstudie wieder. Am ersten Tag zeigte sich bereits nach 20 Minuten nach der ersten Applikation der zu vergleichenden beiden Formulierungen A und B ein deutlich meßbarer Rückgang des transepidermalen Wasserverlustes. Nach 120 Minuten nach der ersten Applikation am ersten Tag war der transepidermale Wasserverlust bei der erfindungsgemäßen Formulierung A geringer als bei der Vergleichsrezeptur B, wobei die Unterschiede im transepidermalen Wasserverlust zwischen den Hautarealen, die mit der erfindungsgemäßen Formulierung A behandelt worden sind und den Hautarealen, die mit der herkömmlichen Vergleichsrezeptur B behandelt wurden, deutlich anhand der Meßwerte erkennbar sind. Diese Aussage verstärkte sich am zweiten Tag und am dritten Tag, so daß die erfindungsgemäße Formulierung A die durch die Behandlung mit der Natronlauge erzeugte Barriereschädigung wesentlich schneller als die herkömmliche Vergleichsrezeptur B heilte.

Die in der vorstehenden Tabelle 1 wiedergegebenen Ergebnisse sind in Figur 6 grafisch dargestellt. Hierin zeigt die untere Kurve (mit 1 gekennzeichnet) den transepidermalen Wasserverlust der nicht geschädigten und nicht behandelten Hautareale. Die gepunktete Kurve (mit 2 gekennzeichnet) gibt den transepidermalen Wasserverlust der geschädigten und anschließend mit der erfindungsgemäßen Formulierung A behandelten Hautareale wieder, während die durchgezogene und mit ausgefüllten Quadraten abgebildete Kurve (mit 3 gekennzeichnet) den transepidermalen Wasserverlust der geschädigten und anschließend mit der herkömmlichen Vergleichsrezeptur B behandelten Hautareale zeigt. Die oberste Kurve (mit 4 gekennzeichnet) bildet den transepidermalen Wasserverlust der geschädigten, aber unbehandelten Hautareale ab.

Von den zuvor bezüglich ihrer Zusammensetzung quantifizierten und bezüglich ihrer Wirksamkeit zu vergleichenden Formulierungen wurde eine Röntgenstrukturanalyse angefertigt. Das Ergebnis dieser Röntgenstrukturanalyse ist in Figur 4 wiedergegeben.

Hierdurch wird eindeutig belegt, daß die erfindungsgemäße Formulierung sowohl einen Peak bei 4,16 Å als auch bei 3,71 Å (obere Kurve) aufweist und somit ortho rhombisch lamellare kristalline Strukturen aufweist, während die konventionelle Vergleichsrezeptur B nur einen einzigen Peak bei 4,16 Å (untere Kurve) zeigt und dementsprechend eine hexagonale lamellare kristalline Struktur besitzt.

Desweiteren wurden die nachfolgend beschriebenen Ausführungsbeispiele 1 bis 12 hergestellt.

### Ausführungsbeispiele

Allgemein ist zu den Ausführungsbeispielen 1 bis 12 anzumerken, daß bei allen Herstellungsverfahren jede der dort beschriebenen Abkühlung mit einer Abkühlrate von 1,5 °C/min ± 0,5 °C/min durchgeführt wurde.

In den Ausführungsbeispielen 1, 2, 4, 7, 8, 9 und 11 wurde ein Lösungsmittel- bzw. Wirkstoffgemisch aus 4-t-Butylcyclohexanol mit Pentylenglycol verwendet. Hierbei handelte es sich um das Handelsprodukt Symsitive, das von der Firma Symrise AG vertrieben wird.

Die bei allen Ausführungsbeispielen beschriebene Inprozesskontrolle wurde unter Verwendung eines Lichtmikroskop (Systemmikroskop Olympus CH2, Model CHT, Nikon Coolpix) unter 100facher Vergrößerung durchgeführt. Hierbei wurde die Homogenität der jeweiligen Dispersion und deren Gleichmäßigkeit visuell beurteilt.

Die in den nachfolgenden Ausführungsbeispielen 1 bis 12 beschriebenen kosmetischen Produkte wurden auf einem Labor-Hochdruckhomogenisator APV Gaulin Micron Lab 40 durchgeführt.

**Ausführungsbeispiel 1**

| *Creme für die überempfindliche, zu Allergien neigende Haut* | | |
|---|---|---|
| Phase | Rohstoff | [%] |
| 1 | Hydriertes Phospholipid | 1,500 |
| 1 | Reiskeim Wachs | 1,000 |
| 1 | Isostearly Isostearate | 3,000 |
| 1 | Pentylene Glycol | 1,250 |
| 1 | Glycerin | 0,750 |
| 2 | Wasser | 17,500 |
| 3 | Moringa Butter | 2,500 |
| 3 | Acrylates/Vinyl Isodecanoate Crosspolymer | 0,270 |
| 3 | Isostearly Isostearate | 8,000 |
| 3 | Olus Oil | 5,000 |
| 4 | Xanthan Gum | 0,100 |
| 4 | Wasser | 54,030 |
| 4 | Pentylene Glycol | 0,750 |
| 4 | Hydroxyethylcellulose | 0,150 |
| 4 | L-Serine | 0,700 |
| 4 | Sodium Polyaspertate | 0,200 |
| 4 | L-Glycine | 0,300 |
| 4 | 1,2 Hexandiol | 1,500 |
| 5 | Natronlauge 30 %ig | 0,30 |
| 6 | 4-t-Butylcyclohexanol, Pentylene Glycol (Symsitive) | 1,200 |
| | Σ | 100,000 |

Phase 1 wurde unter gleichmäßigem Rühren auf 90 °C erwärmt. Anschließend wurde Phase 2 ebenfalls auf 90 °C erwärmt. Bei 90 °C wurde Phase 2 zu der Phase 1 gegeben und anschließend bei 15.000 U/min mittels Ultra Turrax 3 Minuten homogenisiert. Anschließend wurde die Mischung auf 77 °C unter Homogenisation (12.000 U/min mittels Ultra Turrax) abgekühlt und der eingangs beschriebenen Inprozesskontrolle unterzogen.

Die so erstellte Mischung wies eine homogene Dispersion mit gleichmäßig großen Teilchen auf.

Die entstandene Prädispersion wurde mittels Hochdruckhomogenisator feinstdispergiert. Es waren 3 Zyklen bei 800 bar erforderlich. Anschließend wurde das Gemisch unter Rühren auf 35 °C abgekühlt. Das Vorphasengemisch wurde nun bei 300 bar (1 Zyklus) zwangshochdruckhomogenisiert und in einem separaten Gefäß bei 35 °C zwischengelagert. Im Ansatzbehälter wurde nun die Phase 3 unter Rühren auf 50 °C erwärmt und so lange gerührt, bis das Lipid vollständig geschmolzen ist. In einem weiteren Behälter wurde unter Dispergieren die Phase 4 bei 50 °C gerührt, bis eine klare Geldispersion entstanden ist. Die Phase 4 wurde nun zu der Phase 3 gegeben und anschließend bei 50 °C bei 20.000 U/min mittels Ultra Turrax 7 Minuten homogenisiert.

Anschließend wurde die Phase 5 zu der Mischung aus Phase 3+4 gegeben und bei 12.000 U/min mittels Ultra Turrax für 2 Minuten homogenisiert. Nachfolgend wurde die Phase 6 zu der Mischung aus Phase 3+4+5 gegeben und während eines kontinuierlichen Homogenisierungsprozesses bei 12.000 U/min mittels Ultra Turrax auf die Zieltemperatur von 35°C runterhomogenisiert. Dieser Vorgang dauerte 12 Minuten. Anschließend wurde die Prädispersion aus den Phasen 1+2 zu der Mischung aus den Phasen 3+4+5+6 gegeben und bei 20.000 U/min unter kontinuierlichem Rühren 10 Minuten homogenisiert. Abschließend wurde das Gemisch unter kontinuierlichem Homogenisieren bei 12.000 U/min auf 25 °C heruntergekühlt. Dieser Vorgang dauerte 19 Minuten.

**Ausführungsbeispiel 2**

| *Bodycream Lotion für die überempfindliche, zu Allergien neigende Haut* | | |
|---|---|---|
| Phase | Rohstoff | [%] |
| 1 | Hydriertes Phospholipid | 1,200 |
| 1 | Reiskeim Wachs | 1,200 |
| 1 | Isostearly Isostearate | 1,600 |
| 1 | Pentylene Glycol | 1,000 |
| 1 | Glycerin | 1,600 |
| 2 | Wasser | 13,400 |
| 3 | Moringa Öl | 4,000 |
| 3 | Acrylates/Vinyl Isodecanoate Crosspolymer | 0,150 |
| 3 | Isostearly Isostearate | 8,000 |
| 3 | Caprylic/Capric Triglyceride | 15,000 |
| 4 | Xanthan Gum | 0,100 |
| 4 | Wasser | 47,280 |
| 4 | Pentylene Glycol | 1,250 |
| 4 | Hydroxyethylcellulose | 0,150 |
| 4 | L-Serine | 0,700 |
| 4 | Sodium Polyaspertate | 0,200 |
| 4 | L-Glycine | 0,300 |
| 4 | 1,2 Hexandiol | 1,500 |
| 5 | Natronlauge 30 % | 0,17 |
| 6 | 4-t-Butylcyclohexanol, Pentylene Glycol (Symsitive) | 1,200 |
| | Σ | 100,0 |

Phase 1 wurde unter gleichmäßigem Rühren auf 90 °C erwärmt. Anschließend wurde Phase 2 ebenfalls auf 90 °C erwärmt. Bei 90 °C wurde Phase 2 zu der Phase 1 gegeben und anschließend bei 15.000 U/min mittels Ultra Turrax (6 Minuten) homogenisiert. Anschließend wurde die Mischung auf 77 °C unter Homogenisation (12.000 U/min mittels Ultra Turrax) abgekühlt und der eingangs beschriebenen Inprozesskontrolle unterzogen.

Die so erstellte Mischung wies eine homogene Dispersion mit gleichmäßig großen Teilchen auf.

Die entstandene Prädispersion wurde mittels Hochdruckhomogenisator feinstdispergiert. Es waren 5 Zyklen bei 800 bar erforderlich. Anschließend wurde das Gemisch unter Rühren auf 35 °C abgekühlt. Das Vorphasengemisch wurde nun bei 500 bar (1 Zyklus) zwangshochdruckhomogenisiert und in einem separaten Gefäß bei 35 °C zwischengelagert. Im Ansatzbehälter wurde nun die Phase 3 unter Rühren auf 40 °C erwärmt. In einem weiteren Behälter wurde unter Dispergieren die Phase 4 bei 40 °C gerührt, bis eine klare Geldispersion entstanden ist. Die Phase 4 wurde nun zu der Phase 3 gegeben und anschließend bei 40 °C bei 12.000 U/min mittels Ultra Turrax (4 Minuten) homogenisiert.

Anschließend wurde die Phase 5 zu der Mischung aus Phase 3+4 gegeben und bei 10.000 U/min mittels Ultra Turrax für 2 Minuten homogenisiert. Nachfolgend wurde die Phase 6 zu der Mischung aus Phase 3+4+5 gegeben und bei 12.000 U/min mittels Ultra Turrax für weitere 2 Minuten homogenisiert. Nun wurde die Mischung während eines kontinuierlichen Homogenisierungsprozesses bei 12.000 U/min mittels Ultra Turrax auf die Zieltemperatur von 35°C runterhomogenisiert. Dieser Vorgang dauerte 6 Minuten. Anschließend wurde die Prädispersion aus den Phasen 1+2 zu der Mischung aus den Phasen 3+4+5+6 gegeben und bei 15.000 U/min unter kontinuierlichem Rühren 5 Minuten homogenisiert. Abschließend wurde das Gemisch unter kontinuierlichem Homogenisieren bei 12.000 U/min auf 25 °C heruntergekühlt. Dieser Vorgang dauerte 15 Minuten.

**Ausführungsbeispiel 3**

| *Schutzcreme bei Exposition zu extremen Umwelteinflüssen* | | |
|---|---|---|
| Phase | Rohstoff | [%] |
| 1 | Hydriertes Phospholipid | 1,500 |
| 1 | Reiskeim Wachs | 1,000 |
| 1 | Isostearly Isostearate | 3,000 |
| 1 | Pentylene Glycol | 1,250 |
| 1 | Glycerin | 0,750 |
| 2 | Wasser | 17,500 |
| 3 | Moringa Butter | 2,000 |
| 3 | Acrylates/Vinyl Isodecanoate Crosspolymer | 0,350 |
| 3 | Isostearly Isostearate | 4,000 |
| 3 | Tinosorb S (Bis-Ethylhexyloxyphenol Methoxyphenyl Triazine) | 1,500 |
| 3 | Uvinul A Plus (Diethylamino Hydroxybenzoyl Hexyl Benzoate) | 1,500 |
| 3 | Ronacare AP (Bis-Ethylhexyl Hydroxydimethoxy Benzylmalonate) | 1,000 |
| 4 | Wasser | 52,850 |
| 4 | Pentylene Glycol | 0,750 |
| 4 | Hyaluronsäure 400 kDa | 0,400 |
| 4 | Glycerin | 8,000 |
| 4 | L-Glycine | 0,500 |
| 4 | 1,2 Hexandiol | 1,500 |
| 5 | Troxerutin | 0,250 |
| 6 | Natronlauge 30 % | 0,400 |
| | Σ | 100,000 |

Phase 1 wurde unter gleichmäßigem Rühren auf 90 °C erwärmt. Anschließend wurde Phase 2 ebenfalls auf 90 °C erwärmt. Bei 90 °C wurde Phase 2 zu der Phase 1 gegeben und anschließend bei 15.000 U/min mittels Ultra Turrax 3 Minuten homogenisiert. Anschließend wird die Mischung auf 77 °C unter Homogenisation (12.000 U/min mittels Ultra Turrax) abgekühlt und der eingangs beschriebenen Inprozesskontrolle unterzogen.

Die so erstellte Mischung wies eine homogene Dispersion mit gleichmäßig großen Teilchen auf.

Die entstandene Prädispersion wurde mittels Hochdruckhomogenisator feinstdispergiert. Es waren 3 Zyklen bei 800 bar erforderlich. Anschließend wurde das Gemisch unter Rühren auf 35 °C abgekühlt. Das Vorphasengemisch wurde nun bei 300 bar (1 Zyklus) zwangshochdruckhomogenisiert und in einem separaten Gefäß bei 35 °C zwischengelagert. Im Ansatzbehälter wird nun die Phase 3 unter Rühren auf 80 °C erwärmt und so lange gerührt, bis die UV Filter vollständig geschmolzen sind. In einem weiteren Behälter wurde unter Dispergieren die Phase 4 bei 80 °C gerührt, bis eine klare Geldispersion entstanden ist. Die Phase 4 wurde nun zu der Phase 3 gegeben und anschließend bei 80 °C bei 24.000 U/min mittels Ultra Turrax 8 Minuten homogenisiert.

Anschließend wurde die Phase 5 zu der Mischung aus Phase 3+4 gegeben und bei 20.000 U/min mittels Ultra Turrax für 4 Minuten homogenisiert. Nachfolgend wurde die Phase 6 zu der Mischung aus Phase 3+4+5 gegeben und bei 20.000 U/min mittels Ultra Turrax für weitere 5 Minuten homogenisiert. Nun wurde die Mischung während eines kontinuierlichen Homogenisierungsprozesses bei 12.000 U/min mittels Ultra Turrax auf die Zieltemperatur von 35°C runterhomogenisiert. Dieser Vorgang dauerte 30 Minuten. Anschließend wurde die Prädispersion aus den Phasen 1+2 zu der Mischung aus den Phasen 3+4+5+6 gegeben und bei 20.000 U/min unter kontinuierlichem Rühren 5 Minuten homogenisiert. Abschließend wurde das Gemisch unter kontinuierlichem Homogenisieren bei 18.000 U/min auf 25 °C heruntergekühlt. Dieser Vorgang dauerte 17 Minuten.

**Ausführungsbeispiel 4**

| *Pflegecreme zur begleitenden adjuvanten Behandlung bei Neurodermitis erkrankter Haut* | | |
|---|---|---|
| Phase | Rohstoff | [%] |
| 1 | Hydriertes Phospholipid | 1,800 |
| 1 | Reiskeim Wachs | 1,800 |
| 1 | Isostearly Isostearate | 2,400 |
| 1 | Pentylene Glycol | 1,500 |
| 1 | Glycerin | 1,800 |
| 2 | Wasser | 20,700 |
| 3 | Moringa Butter | 6,000 |
| 3 | Sodium Carbomer | 0,180 |
| 3 | Boswellic Acid 20 % | 2,500 |
| 3 | Caprylic/Capric Triglyceride | 6,000 |
| 4 | Xanthan Gum | 0,100 |
| 4 | Wasser | 45,170 |
| 4 | Pentylene Glycol | 1,000 |
| 4 | Hydroxyethylcellulose | 0,150 |
| 4 | L-Serine | 0,700 |
| 4 | Niacinamide | 4,000 |
| 4 | Sodium Polyaspertate | 0,200 |
| 4 | L-Glycine | 0,300 |
| 4 | 1,2 Hexandiol | 1,500 |
| 4 | Butylene Glycol, Pentylene Glycol, Hydroxyphenyl Propamidobenzoic Acid (Symcalmin) | 1,000 |
| 5 | 4-t-Butylcyclohexanol, Pentylene Glycol (Symsitive) | 1,200 |
| | Σ | 100,000 |

Phase 1 wurde unter gleichmäßigem Rühren auf 90 °C erwärmt. Anschließend wurde Phase 2 ebenfalls auf 90 °C erwärmt. Bei 90 °C wurde Phase 2 zu der Phase 1 gegeben und anschließend bei 15.000 U/min mittels Ultra Turrax 8 Minuten homogenisiert. Anschließend wurde die Mischung auf 77 °C unter Homogenisation (12.000 U/min mittels Ultra Turrax) abgekühlt und der eingangs beschriebenen Inprozesskontrolle unterzogen.

Die so erstellte Mischung wies eine inhomogene Dispersion mit ungleichmäßig großen Teilchen auf.

Von daher wurde die Mischung erneut unter Rühren auf 90 °C erwärmt und anschließend bei 18.000 U/min mittels Ultra Turrax 5 Miniuten homogenisiert und die Mischung auf 77 °C unter Homogenisation (18.000 U/min mittels Ultra Turrax) abgekühlt.

Die erneute Inprozesskontrolle ergab eine homogene Dispersion mit gleichmäßig großen Teilchen.

Die entstandene Prädispersion wurde mittels Hochdruckhomogenisator feinstdispergiert. Es waren 5 Zyklen bei 800 bar erforderlich. Anschließend wurde das Gemisch unter Rühren auf 35 °C abgekühlt. Das Vorphasengemisch wurde nun bei 600 bar (1 Zyklus) zwangshochdruckhomogenisiert und in einem separaten Gefäß bei 35 °C zwischengelagert. Im Ansatzbehälter wurde nun die Phase 3 unter Rühren auf 50 °C erwärmt. In einem weiteren Behälter wurde unter Dispergieren die Phase 4 bei 50 °C gerührt, bis eine klare Geldispersion entstanden ist. Die Phase 4 wurde nun zu der Phase 3 gegeben und anschließend bei 50 °C bei 18.000 U/min mittels Ultra Turrax 3 Minuten homogenisiert.

Anschließend wurde die Phase 5 zu der Mischung aus Phase 3+4 gegeben und bei 20.000 U/min mittels Ultra Turrax 5 Minuten homogenisiert. Nun wurde die Mischung während eines kontinuierlichen Homogenisierungsprozesses bei 15.000 U/min mittels Ultra Turrax auf die Zieltemperatur von 35°C runterhomogenisiert. Dieser Vorgang dauerte 14 Minuten. Anschließend wurde die Prädispersion aus den Phasen 1+2 zu der Mischung aus den Phasen 3+4+5 gegeben und bei 20.000 U/min unter kontinuierlichem Rühren 7 Minuten homogenisiert. Abschließend wurde das Gemisch unter kontinuierlichem Homogenisieren bei 20.000 U/min auf 25 °C heruntergekühlt. Dieser Vorgang dauerte 18 Minuten.

**Ausführungsbeispiel 5**

| *Sonnenschutz für zu PLD (Polymorphe Lichtdermatose) neigender Haut* | | |
|---|---|---|
| Phase | Rohstoff | [%] |
| 1 | Hydriertes Phospholipid | 1,500 |
| 1 | Ilex Paraguariensis Blatt Wachs | 1,000 |
| 1 | Caprylic/Capric Triglyceride | 2,000 |
| 1 | Pentylene Glycol | 1,500 |
| 1 | Glycerin | 1,000 |
| 2 | Wasser | 20,700 |
| 3 | Octocrylene | 7,000 |
| 3 | PVP/ Eicosene Copolymer | 0,100 |
| 3 | Acrylates/Vinyl Isodecanoate Crosspolymer | 0,350 |
| 3 | Tinosorb S (Bis-Ethylhexyloxyphenol Methoxyphenyl Triazine) | 3,800 |
| 3 | Uvinul A Plus (Diethylamino Hydroxybenzoyl Hexyl Benzoate) | 3,800 |
| 3 | Ronacare AP (Bis-Ethylhexyl Hydroxydimethoxy Benzylmalonate) | 1,000 |
| 3 | C12-15 Alkyl Benzoate | 2,000 |
| 3 | Dicaprylyl Carbonate | 4,000 |
| 4 | Xanthan Gum | 0,100 |
| 4 | Wasser | 47,100 |
| 4 | Pentylene Glycol | 1,000 |
| 4 | Hydroxyethylcellulose | 0,150 |
| 4 | 1,2 Hexandiol | 1,500 |
| 5 | Natronlauge 30 % | 0,400 |
| | Σ | 100,000 |

Phase 1 wurde unter gleichmäßigem Rühren auf 86 °C erwärmt. Anschließend wurde Phase 2 ebenfalls auf 86 °C erwärmt. Bei 86 °C wurde Phase 2 zu der Phase 1 gegeben und anschließend bei 15.000 U/min mittels Ultra Turrax 4 Minuten homogenisiert. Anschließend wurde die Mischung auf 75 °C unter Homogenisation (10.000 U/min mittels Ultra Turrax) abgekühlt und der eingangs beschriebenen Inprozesskontrolle unterzogen.

Die so erstellte Mischung wies eine homogene Dispersion mit gleichmäßig großen Teilchen auf.

Die entstandene Prädispersion wurde mittels Hochdruckhomogenisator feinstdispergiert. Es waren 3 Zyklen bei 800 bar erforderlich. Anschließend wurde das Gemisch unter Rühren auf 35 °C abgekühlt. Das Vorphasengemisch wurde nun bei 300 bar (1 Zyklus) zwangshochdruckhomogenisiert und in einem separaten Gefäß bei 35 °C zwischengelagert. Im Ansatzbehälter wurde nun die Phase 3 unter Rühren auf 80 °C erwärmt und so lange gerührt, bis die UV Filter vollständig geschmolzen sind. In einem weiteren Behälter wurde unter Dispergieren die Phase 4 bei 80 °C gerührt, bis eine klare Geldispersion entstanden ist. Die Phase 4 wurde nun zu der Phase 3 gegeben und anschließend bei 80 °C bei 24.000 U/min mittels Ultra Turrax 10 Minuten homogenisiert.

Anschließend wurde die Phase 5 zu der Mischung aus Phase 3+4 gegeben und bei 18.000 U/min mittels Ultra Turrax für 5 Minuten homogenisiert. Nun wurde die Mischung während eines kontinuierlichen Homogenisierungsprozesses bei 24.000 U/min mittels Ultra Turrax auf die Zieltemperatur von 35°C runterhomogenisiert. Dieser Vorgang dauerte 34 Minuten. Anschließend wurde die Prädispersion aus den Phasen 1+2 zu der Mischung aus den Phasen 3+4+5 gegeben und bei 24.000 U/min unter kontinuierlichem Rühren 7 Minuten homogenisiert. Abschließend wurde das Gemisch unter kontinuierlichem Homogenisieren bei 15.000 U/min auf 25 °C heruntergekühlt. Dieser Vorgang dauerte 16 Minuten.

**Ausführungsbeispiel 6**

| *Pflegelippencreme bei Herpesneigung* | | |
|---|---|---|
| Phase | Rohstoff | [%] |
| 1 | Hydriertes Phospholipid | 2,000 |
| 1 | Carnauba Wachs | 2,000 |
| 1 | Isostearly Isostearate | 2,500 |
| 1 | Pentylene Glycol | 1,800 |
| 1 | Glycerin | 3,000 |
| 2 | Wasser | 23,200 |
| 3 | Moringa Butter | 2,000 |
| 3 | Acrylates/Vinyl Isodecanoate Crosspolymer | 0,150 |
| 3 | Isostearly Isostearate | 4,000 |
| 3 | Tinosorb S (Bis-Ethylhexyloxyphenol Methoxyphenyl Triazine) | 2,800 |
| 3 | Uvinul A Plus (Diethylamino Hydroxybenzoyl Hexyl Benzoate) | 2,800 |
| 3 | Ronacare AP (Bis-Ethylhexyl Hydroxydimethoxy Benzylmalonate) | 1,000 |
| 4 | Wasser | 42,530 |
| 4 | Xanthan Gum | 0,100 |
| 4 | Pentylene Glycol | 0,200 |
| 4 | Glycerin | 8,000 |
| 4 | 1,2 Hexandiol | 1,500 |
| 5 | Troxerutin | 0,250 |
| 6 | Natronlauge 30 % | 0,17 |
| | Σ | 100,000 |

Phase 1 wurde unter gleichmäßigem Rühren auf 92 °C erwärmt. Anschließend wurde Phase 2 ebenfalls auf 92 °C erwärmt. Bei 92 °C wurde Phase 2 zu der Phase 1 gegeben und anschließend bei 15.000 U/min mittels Ultra Turrax 12 Minuten homogenisiert. Anschließend wird die Mischung auf 77 °C unter Homogenisation (13.000 U/min mittels Ultra Turrax) abgekühlt und der eingangs beschriebenen Inprozesskontrolle unterzogen.

Die so erstellte Mischung wies eine inhomogene Dispersion mit ungleichmäßig großen Teilchen auf.

Von daher wurde die Mischung erneut unter Rühren auf 92 °C erwärmt und anschließend bei 13.000 U/min mittels Ultra Turrax 9 Minuten homogenisiert und die Mischung auf 77 °C unter Homogenisation (18.000 U/min mittels Ultra Turrax) abgekühlt. Die erneute Inprozesskontrolle ergab eine homogene Dispersion mit gleichmäßig großen Teilchen.

Die entstandene Prädispersion wurde mittels Hochdruckhomogenisators feinstdispergiert. Es waren 6 Zyklen bei 800 bar erforderlich. Anschließend wird das Gemisch unter Rühren auf 35 °C abgekühlt. Das Vorphasengemisch wurde nun bei 700 bar (1 Zyklus) zwangshochdruckhomogenisiert und in einem separaten Gefäß bei 35 °C zwischengelagert.

Im Ansatzbehälter wurde nun die Phase 3 unter Rühren auf 80 °C erwärmt und so lange gerührt, bis die UV Filter vollständig geschmolzen sind. In einem weiteren Behälter wurde unter Dispergieren die Phase 4 bei 80 °C gerührt, bis eine klare Geldispersion entstanden war. Phase 5 wurde nun unter kontinuierlichem Rühren zu Phase 4 gegeben und so lange gerührt bis eine klare Dispersion entstanden war. Die Phase 4+5 wurde nun zu der Phase 3 gegeben und anschließend bei 80 °C bei 18.000 U/min mittels Ultra Turrax 5 Minuten homogenisiert.

Anschließend wurde die Phase 6 zu der Mischung aus Phase 3+4+5 gegeben und bei 24.000 U/min mittels Ultra Turrax 5 Minuten homogenisiert. Nun wurde die Mischung während eines kontinuierlichen Homogenisierungsprozesses bei 24.000 U/min mittels Ultra Turrax auf die Zieltemperatur von 35°C runterhomogenisiert. Dieser Vorgang dauerte 19 Minuten. Anschließend wurde die Prädispersion aus den Phasen 1+2 zu der Mischung aus den Phasen 3+4+5+6 gegeben und bei 24.000 U/min unter kontinuierlichem Rühren 11 Minuten homogenisiert. Abschließend wurde das Gemisch unter kontinuierlichem Homogenisieren bei 18.000 U/min auf 25 °C heruntergekühlt. Dieser Vorgang dauerte 19 Minuten.

Auch von diesen Ausführungsbeispielen 1 bis 6 wurden unter den vorstehend beschriebenen Bedingungen Röntgenstrukturanalysen durchgeführt. Das Ergebnis dieser Röntgenstrukturanalyse ist in Figur 5 zusammengefaßt wiedergegeben.

Hierdurch wird eindeutig bewiesen, daß die erfindungsgemäßen Formulierungen gemäß der Ausführungsbeispiele 1 bis 6 alle sowohl einen Peak bei 4,16 Å als auch bei 3,71 Å aufweisen und somit alle Formulierungen ortho rhombisch lamellare kristalline Strukturen enthalten.

**Ausführungsbeispiel 7**

| *Creme für die zu Hyper Reaktivität neigenden Haut* | | |
|---|---|---|
| Phase | Rohstoff | [%] |
| 1 | Hydriertes Phospholipid | 1,750 |
| 1 | Carnauba Wachs | 0,950 |
| 1 | Ficus Cerifera Wachs | 0,620 |
| 1 | Isostearly Isostearate | 4,500 |
| 1 | Pentylene Glycol | 1,250 |
| 1 | Glycerin 99,5 % | 0,750 |
| 2 | Wasser | 16,180 |
| 3 | Moringa Butter | 5,000 |
| 3 | Acrylates/Vinyl Isodecanoate Crosspolymer | 0,280 |
| 3 | Isostearly Isostearate | 1,000 |
| 3 | Olus Oil | 5,000 |
| 4 | Xanthan Gum | 0,100 |
| 4 | Wasser | 52,220 |
| 4 | Pentylene Glycol | 0,750 |
| 4 | Hydroxyethylcellulose | 0,150 |
| 4 | Glycerin 99,5 % | 6,000 |
| 4 | Sodium PCA Lsg. 50 % | 0,200 |
| 4 | L-Glycine | 0,300 |
| 4 | 1,2 Hexandiol | 1,500 |
| 5 | Natronlauge 30 %ig | 0,300 |
| 6 | 4-T-Butylcyclohexanol, Pentylene Glycol (Symsitive) | 1,200 |
| | Σ | 100,000 |

Phase 1 wurde unter gleichmäßigem Rühren auf 85 °C erwärmt. Anschließend wurde Phase 2 ebenfalls auf 90 °C erwärmt. Bei 85 °C wurde Phase 2 zu der Phase 1 gegeben und anschließend bei 15.000 U/min mittels Ultra Turrax 5 Minuten homogenisiert. Anschließend wurde die Mischung auf 78 °C unter Homogenisation (12.000 U/min mittels Ultra Turrax) abgekühlt und der eingangs beschriebenen Inprozesskontrolle unterzogen.

Die so erstellte Mischung wies eine homogene Dispersion mit gleichmäßig großen Teilchen auf.

Die entstandene Prädispersion wurde mittels Hochdruckhomogenisator feinstdispergiert. Es sind 3 Zyklen bei 800 bar erforderlich. Anschließend wurde das Gemisch unter Rühren und gleichmäßiger Homogenisation bei 9.000 U/min (Ultra Turrax) auf 35 °C abgekühlt. Das Vorphasengemisch wurde nun bei 350 bar (1 Zyklus) zwangshochdruckhomogenisiert und in einem separaten Gefäß bei 35 °C zwischengelagert. Im Ansatzbehälter wurde nun die Phase 3 unter Rühren auf 55 °C erwärmt und so lange gerührt, bis das Lipid vollständig geschmolzen war. In einem weiteren Behälter wurde unter Dispergieren die Phase 4 bei 55 °C gerührt, bis eine klare Geldispersion entstanden war. Die Phase 4 wurde nun zu der Phase 3 gegeben und anschließend bei 50 °C bei 20.000 U/min mittels Ultra Turrax 6 Minuten homogenisiert.

Anschließend wurde die Phase 5 zu der Mischung aus Phase 3+4 gegeben und bei 12.000 U/min mittels Ultra Turrax für 3 Minuten homogenisiert. Nachfolgend wurde die Phase 6 zu der Mischung aus Phase 3+4+5 gegeben und während eines kontinuierlichen Homogenisierungsprozesses bei 12.000 U/min mittels Ultra Turrax auf die Zieltemperatur von 35°C runterhomogenisiert. Dieser Vorgang dauerte 16 Minuten. Anschließend wurde die Prädispersion aus den Phasen 1+2 zu der Mischung aus den Phasen 3+4+5+6 gegeben und bei 22.000 U/min unter kontinuierlichem Rühren 10 Minuten homogenisiert. Abschließend wurde das Gemisch unter kontinuierlichem Homogenisieren bei 12.000 U/min auf 25 °C heruntergekühlt. Dieser Vorgang dauerte 21 Minuten.

**Ausführungsbeispiel 8**

| *Creme für die Vorbeugung von hypertropher Narbenbildung* | | |
|---|---|---|
| Phase | Rohstoff | [%] |
| 1 | Hydriertes Phospholipid | 1,800 |
| 1 | Bambusoideae Wachs | 0,750 |
| 1 | Carnauba Wachs | 1,050 |
| 1 | Isostearly Isostearate | 3,500 |
| 1 | Pentylene Glycol | 1,500 |
| 1 | Glycerin 99,5 % | 1,000 |
| 2 | Wasser | 20,400 |
| 3 | Moringa Butter | 2,000 |
| 3 | Eusolex 9020 | 5,000 |
| 3 | Tinosorb S | 3,800 |
| 3 | Octocrylene | 7,000 |
| 3 | Uvinul A Plus | 3,800 |
| 3 | TiO2 | 1,500 |
| 3 | Acrylates/Vinyl Isodecanoate Crosspolymer | 0,250 |
| 3 | Finsolv TN | 6,000 |
| 3 | Olus Oil | 3,000 |
| 4 | Xanthan Gum | 0,100 |
| 4 | Wasser | 24,480 |
| 4 | Pentylene Glycol | 0,750 |
| 4 | Hydroxyethylcellulose | 0,150 |
| 4 | Glycerin 99,5 % | 3,000 |
| 4 | Ectoin | 1,000 |
| 4 | Madecassoside | 0,200 |
| 4 | 1,2 Hexandiol | 1,500 |
| 5 | Natronlauge 30 %ig | 0,270 |
| 6 | 4-T-Butylcyclohexanol, Pentylene Glycol (Symsitive) | 1,200 |
| 6 | Polyhexan (Water, Snail Secretion Filtrate, Propylene Glycol, Glycerine, Glycosaminoglycans, Allantoin, Hydrolyzed Collagen, Pyridoxine HCL, PoxEthanol) | 5,000 |
| | Σ | 100,000 |

Phase 1 wurde unter gleichmäßigem Rühren auf 85 °C erwärmt. Anschließend wurde Phase 2 ebenfalls auf 90 °C erwärmt. Bei 85 °C wurde Phase 2 zu der Phase 1 gegeben und anschließend bei 15.000 U/min mittels Ultra Turrax 5 Minuten homogenisiert. Anschließend wurde die Mischung auf 78 °C unter Homogenisation (12.000 U/min mittels Ultra Turrax) abgekühlt und der eingangs beschriebenen Inprozesskontrolle unterzogen.

Die so erstellte Mischung wies eine homogene Dispersion mit gleichmäßig großen Teilchen auf.

Die entstandene Prädispersion wurde mittels Hochdruckhomogenisator feinstdispergiert. Es waren 3 Zyklen bei 800 bar erforderlich. Anschließend wurde das Gemisch unter Rühren und gleichmäßiger Homogenisation bei 9.000 U/min (Ultra Turrax) auf 35 °C abgekühlt. Das Vorphasengemisch wurde nun bei 350 bar (1 Zyklus) zwangshochdruckhomogenisiert und in einem separaten Gefäß bei 35 °C zwischengelagert. Im Ansatzbehälter wurde nun die Phase 3 unter Rühren auf 85 °C erwärmt und so lange gerührt, bis die lipidischen UV Filter vollständig geschmolzen waren. In einem weiteren Behälter wurde unter Dispergieren die Phase 4 bei 85 °C gerührt, bis eine klare Geldispersion entstanden ist. Die Phase 4 wurde nun zu der Phase 3 gegeben und anschließend bei 85 °C bei 20.000 U/min mittels Ultra Turrax 6 Minuten homogenisiert.

Anschließend wurde die Phase 5 zu der Mischung aus Phase 3+4 gegeben und bei 18.000 U/min mittels Ultra Turrax für 3 Minuten homogenisiert. Nun wurde die Dispersion aus den Phasen 3+4+5 bei 18.000 U/min mittels Ultra Turrax auf 50 °C abgekühlt. Nachfolgend wurde die Phase 6 zu der Mischung aus Phase 3+4+5 gegeben und während eines kontinuierlichen Homogenisierungsprozesses bei 18.000 U/min mittels Ultra Turrax auf die Zieltemperatur von 35°C runterhomogenisiert. Dieser Vorgang dauerte 20 Minuten. Anschließend wurde die Prädispersion aus den Phasen 1+2 zu der Mischung aus den Phasen 3+4+5+6 gegeben und bei 24.000 U/min unter kontinuierlichem Rühren 14 Minuten homogenisiert. Abschließend wurde das Gemisch unter kontinuierlichem Homogenisieren bei 18.000 U/min auf 25 °C heruntergekühlt. Dieser Vorgang dauerte 17 Minuten.

**Ausführungsbeispiel 9**

| *Creme für die Vorbeugung actinischer Keratosen* | | |
|---|---|---|
| Phase | Rohstoff | [%] |
| 1 | Hydriertes Phospholipid | 1,800 |
| 1 | Carnauba Wachs | 1,200 |
| 1 | Isostearly Isostearate | 3,500 |
| 1 | Pentylene Glycol | 1,500 |
| 1 | Glycerin 99,5 % | 1,000 |
| 2 | Wasser | 21,000 |
| 3 | Eusolex 9020 | 5,000 |
| 3 | Tinosorb S | 3,800 |
| 3 | Octocrylene | 7,000 |
| 3 | Uvinul A Plus | 3,800 |
| 3 | Ti02 | 1,500 |
| 3 | Ronacare AP | 1,000 |
| 3 | Acrylates/Vinyl Isodecanoate Crosspolymer | 0,260 |
| 3 | Finsolv TN | 9,000 |
| 3 | Olus Oil | 3,000 |
| 4 | Xanthan Gum | 0,100 |
| 4 | Wasser | 28,310 |
| 4 | Pentylene Glycol | 0,750 |
| 4 | Hydroxyethylcellulose | 0,150 |
| 4 | Glycerin 99,5 % | 3,000 |
| 4 | Ronacare Troxerutin | 0,250 |
| 4 | Spectrastate PHL | 1,600 |
| 5 | Natronlauge 30 %ig | 0,280 |
| 6 | 4-T-Butylcyclohexanol, Pentylene Glycol (Symsitive) | 1,200 |
| | Σ | 100,000 |

Phase 1 wurde unter gleichmäßigem Rühren auf 85 °C erwärmt. Anschließend wurde Phase 2 ebenfalls auf 90 °C erwärmt. Bei 85 °C wurde Phase 2 zu der Phase 1 gegeben und anschließend bei 15.000 U/min mittels Ultra Turrax 5 Minuten homogenisiert. Anschließend wurde die Mischung auf 78 °C unter Homogenisation (12.000 U/min mittels Ultra Turrax) abgekühlt und der eingangs beschriebenen Inprozesskontrolle unterzogen.

Die so erstellte Mischung wies eine homogene Dispersion mit gleichmäßig großen Teilchen auf.

Die entstandene Prädispersion wurde mittels Hochdruckhomogenisator feinstdispergiert. Es sind 3 Zyklen bei 800 bar erforderlich. Anschließend wurde das Gemisch unter Rühren und gleichmäßiger Homogenisation bei 9.000 U/min (Ultra Turrax) auf 35 °C abgekühlt. Das Vorphasengemisch wurde nun bei 350 bar (1 Zyklus) zwangshochdruckhomogenisiert und in einem separaten Gefäß bei 35 °C zwischengelagert.

Im Ansatzbehälter wurde nun die Phase 3 unter Rühren auf 85 °C erwärmt und so lange gerührt, bis die lipidischen UV Filter vollständig geschmolzen waren. In einem weiteren Behälter wurde unter Dispergieren die Phase 4 bei 85 °C gerührt, bis eine klare, gelbliche Geldispersion entstanden ist. Die Phase 4 wurde nun zu der Phase 3 gegeben und anschließend bei 85 °C bei 18.000 U/min mittels Ultra Turrax 5 Minuten homogenisiert.

Anschließend wurde die Phase 5 zu der Mischung aus Phase 3+4 gegeben und bei 18.000 U/min mittels Ultra Turrax für 5 Minuten homogenisiert.

Nun wurde die Dispersion aus den Phasen 3+4+5 bei 18.000 U/min mittels Ultra Turrax auf 50 °C abgekühlt. Nachfolgend wurde die Phase 6 zu der Mischung aus Phase 3+4+5 gegeben und während eines kontinuierlichen Homogenisierungsprozesses bei 18.000 U/min mittels Ultra Turrax auf die Zieltemperatur von 35°C homogenisiert. Dieser Vorgang dauerte 17 Minuten. Anschließend wurde die Prädispersion aus den Phasen 1+2 zu der Mischung aus den Phasen 3+4+5+6 gegeben und bei 20.000 U/min unter kontinuierlichem Rühren 16 Minuten homogenisiert. Abschließend wurde das Gemisch unter kontinuierlichem Homogenisieren bei 18.000 U/min auf 25 °C heruntergekühlt. Dieser Vorgang dauerte 14 Minuten.

**Ausführungsbeispiel 10**

| *Pflege Creme für die Vorbeugung lichtinduzierter Hautalterungserscheinungen* | | |
|---|---|---|
| **Phase** | **Rohstoff** | [%] |
| 1 | Hydriertes Phospholipid | 1,200 |
| 1 | Carnauba Wachs | 0,800 |
| 1 | Isostearly Isostearate | 2,400 |
| 1 | Pentylene Glycol | 1,000 |
| 1 | Glycerin 99,5 % | 0,600 |
| 2 | Wasser | 14,000 |
| 3 | Moringa Butter | 1,000 |
| 3 | Finsolv TN | 2,500 |
| 3 | Crodamol isis-LQ-(MV) | 1,000 |
| 3 | Myritol 312 | 3,000 |
| 3 | Tinosorb S | 1,500 |
| 3 | Uvinul A Plus | 1,500 |
| 3 | Stabylen 30 | 0,250 |
| 3 | Smartgel P110 | 0,300 |
| 3 | Amaze XT | 0,100 |
| 4 | Osmosewasser | 49,486 |
| 4 | RonaCare (R) Troxerutin | 0,250 |
| 4 | Glycerin 99% Ph.Eur. wasserfrei | 8,000 |
| 4 | Raya Hyaluron LMW (50 k Da) | 0,100 |
| 4 | Hydrolite-5 | 1,000 |
| 5 | Osmosewasser | 5,000 |
| 5 | Niacinamide PC | 2,000 |
| 6 | Hydrolite-6 | 0,300 |
| 7 | Spectrastat (TM) PHL | 1,600 |
| 8 | RonaCare AP | 1,000 |
| 9 | Natriumhydroxid 32%ige Lösung | 0,114 |
| | | **100,000** |

Phase 1 wurde unter gleichmäßigem Rühren auf 85 °C erwärmt. Anschließend wurde Phase 2 ebenfalls auf 90 °C erwärmt. Bei 85 °C wurde Phase 2 zu der Phase 1 gegeben und anschließend bei 15.000 U/min mittels Ultra Turrax 5 Minuten homogenisiert. Anschließend wurde die Mischung auf 78 °C unter Homogenisation (12.000 U/min mittels Ultra Turrax) abgekühlt und der eingangs beschriebenen Inprozesskontrolle unterzogen.

Die so erstellte Mischung wies eine homogene Dispersion mit gleichmäßig großen Teilchen auf.

Die entstandene Prädispersion wurde mittels Hochdruckhomogenisator feinstdispergiert. Es waren 3 Zyklen bei 800 bar erforderlich. Anschließend wurde das Gemisch unter Rühren und gleichmäßiger Homogenisation bei 9.000 U/min (Ultra Turrax) auf 35 °C abgekühlt. Das Vorphasengemisch wurde nun bei 350 bar (1 Zyklus) zwangshochdruckhomogenisiert und in einem separaten Gefäß bei 35 °C zwischengelagert.

Im Ansatzbehälter wurde nun die Phase 3 unter Rühren auf 85 °C erwärmt und so lange gerührt, bis die lipidischen UV Filter vollständig geschmolzen waren. In einem weiteren Behälter wurde unter Dispergieren die Phase 4 bei 85 °C gerührt, bis eine klare, gelbliche Geldispersion entstanden war. Die Phase 4 wurde nun zu der Phase 3 gegeben und anschließend bei 85 °C bei 18.000 U/min mittels Ultra Turrax 5 Minuten homogenisiert.

Anschließend wurde die Phase 5 zu der Mischung aus Phase 3+4 gegeben und bei 18.000 U/min mittels Ultra Turrax für 5 Minuten homogenisiert. Nun wurde die Dispersion aus den Phasen 3+4+5 bei 18.000 U/min mittels Ultra Turrax auf 50 °C abgekühlt. Nachfolgend wurde die Phase 6 zu der Mischung aus Phase 3+4+5 gegeben und während eines kontinuierlichen Homogenisierungsprozesses bei 18.000 U/min mittels Ultra Turrax auf die Zieltemperatur von 35°C homogenisiert. Dieser Vorgang dauerte 17 Minuten. Anschließend wurde die Prädispersion aus den Phasen 1+2 zu der Mischung aus den Phasen 3+4+5+6 gegeben und bei 20.000 U/min unter kontinuierlichem Rühren 16 Minuten homogenisiert. Abschließend wird das Gemisch unter kontinuierlichem Homogenisieren bei 18.000 U/min auf 25 °C heruntergekühlt. Dieser Vorgang dauerte 14 Minuten.

**Ausführungsbeispiel 11**

| *Lichtschutz Creme für die zu Polymorpher Lichtdermatose neigender Haut* | | |
|---|---|---|
| Phase | Rohstoff | [%] |
| 1 | Hydriertes Phospholipid | 1,500 |
| 1 | Carnauba Wachs | 1,500 |
| 1 | Isostearly Isostearate | 2,900 |
| 1 | Pentylene Glycol | 1,250 |
| 1 | Glycerin 99,5 % | 0,600 |
| 2 | Wasser | 17,200 |
| 3 | Eusolex 9020 | 3,500 |
| 3 | Tinosorb S | 2,500 |
| 3 | Octocrylene | 10,000 |
| 3 | Uvinul A Plus | 2,500 |
| 3 | Ti02 | 2,500 |
| 3 | Acrylates/Vinyl Isodecanoate Crosspolymer | 0,260 |
| 3 | Finsolv TN | 5,000 |
| 3 | Olus Oil | 5,000 |
| 4 | Xanthan Gum | 0,100 |
| 4 | Wasser | 34,260 |
| 4 | Hydroxyethylcellulose | 0,150 |
| 4 | Glycerin 99,5 % | 5,000 |
| 4 | Ronacare Troxerutin | 0,250 |
| 4 | Spectrastate PHL | 1,600 |
| 5 | Natronlauge 30 %ig | 0,280 |
| 6 | 4-T-Butylcyclohexanol, Pentylene Glycol (Symsitive) | 1,200 |
| 6 | Tetrahydrocurcuminoids (THC) | 0,200 |
| 6 | Pentylene Glycol | 0,750 |
| | Σ | 100,000 |

Phase 1 wurde unter gleichmäßigem Rühren auf 85 °C erwärmt. Anschließend wurde Phase 2 ebenfalls auf 90 °C erwärmt. Bei 85 °C wurde Phase 2 zu der Phase 1 gegeben und anschließend bei 15.000 U/min mittels Ultra Turrax 5 Minuten homogenisiert. Anschließend wurde die Mischung auf 78 °C unter Homogenisation (12.000 U/min mittels Ultra Turrax) abgekühlt und der eingangs beschriebenen Inprozesskontrolle unterzogen.

Die so erstellte Mischung wies eine homogene Dispersion mit gleichmäßig großen Teilchen auf.

Die entstandene Prädispersion wurde mittels Hochdruckhomogenisator feinstdispergiert. Es waren 3 Zyklen bei 800 bar erforderlich. Anschließend wurde das Gemisch unter Rühren und gleichmäßiger Homogenisation bei 9.000 U/min (Ultra Turrax) auf 35 °C abgekühlt. Das Vorphasengemisch wurde nun bei 350 bar (1 Zyklus) zwangshochdruckhomogenisiert und in einem separaten Gefäß bei 35 °C zwischengelagert.

Im Ansatzbehälter wurde nun die Phase 3 unter Rühren auf 85 °C erwärmt und so lange gerührt, bis die lipidischen UV Filter vollständig geschmolzen waren. In einem weiteren Behälter wurde unter Dispergieren die Phase 4 bei 85 °C gerührt, bis eine klare, gelbliche Geldispersion entstanden war. Die Phase 4 wurde nun zu der Phase 3 gegeben und anschließend bei 85 °C bei 16.000 U/min mittels Ultra Turrax 5 Minuten homogenisiert.

Anschließend wurde die Phase 5 zu der Mischung aus Phase 3+4 gegeben und bei 18.000 U/min mittels Ultra Turrax für 5 Minuten homogenisiert. Nun wurde die Dispersion aus den Phasen 3+4+5 bei 16.000 U/min mittels Ultra Turrax auf 45 °C abgekühlt. Nachfolgend wurde die Phase 6 bei 45 °C unter rühren zur vollständigen Lösung gebracht. Anschließend wurde diese Phase zu der Mischung aus Phase 3+4+5 gegeben und während eines kontinuierlichen Homogenisierungsprozesses bei 16.000 U/min mittels Ultra Turrax auf die Zieltemperatur von 35°C homogenisiert. Dieser Vorgang dauerte 17 Minuten. Anschließend wurde die Prädispersion aus den Phasen 1+2 zu der Mischung aus den Phasen 3+4+5+6 gegeben und bei 16.000 U/min unter kontinuierlichem Rühren 16 Minuten homogenisiert. Abschließend wurde das Gemisch unter kontinuierlichem Homogenisieren bei 16.000 U/min auf 25 °C heruntergekühlt. Dieser Vorgang dauerte 12 Minuten.

**Ausführungsbeispiel 12**

| *Pflege Creme für die überempfindliche, zu Trockenheit und Irritationen neigenden Haut* | | |
|---|---|---|
| **Phase** | **Rohstoff** | [%] |
| 1 | Hydriertes Phospholipid | 1,500 |
| 1 | Carnauba Wachs | 1,000 |
| 1 | Isostearly Isostearate | 3,000 |
| 1 | Pentylene Glycol | 1,250 |
| 1 | Glycerin 99,5 % | 0,750 |
| 2 | Wasser | 17,500 |
| 3 | Crodamol isis-LQ-(MV) | 2,000 |
| 3 | Moringa Butter | 1,500 |
| 3 | Cetiol SB 45 | 4,500 |
| 4 | Cegesoft PS6 | 7,500 |
| 4 | Soothex 20 CQ U/A | 2,500 |
| 5 | Antaron V-220 F | 0,100 |
| 5 | Stabylen 30 | 0,270 |
| 5 | Amaze XT | 0,050 |
| 6 | Osmosewasser | 39,870 |
| 6 | Glycerin 99% Ph.Eur. wasserfrei | 8,000 |
| 6 | Niacinamide PC | 2,000 |
| 6 | Natrosol 250GR | 0,150 |
| 6 | Symcalmin | 1,000 |
| 7 | Symsitive 1609 | 0,400 |
| 8 | Osmosewasser | 2,000 |
| 8 | L-Serin | 0,700 |
| 8 | Glycine (L) | 0,300 |
| 8 | Sodium Polyaspartate C-LC/SD-PC | 0,100 |
| 9 | Spectrastat (TM) PHL | 1,600 |
| 9 | Hydrolite-6 | 0,300 |
| 10 | Natriumhydroxid 32%ige Lösung | 0,160 |
| | | **100,000** |

Phase 1 wurde unter gleichmäßigem Rühren auf 85 °C erwärmt. Anschließend wurde Phase 2 ebenfalls auf 90 °C erwärmt. Bei 85 °C wurde Phase 2 zu der Phase 1 gegeben und anschließend bei 15.000 U/min mittels Ultra Turrax 5 Minuten homogenisiert. Anschließend wurde die Mischung auf 78 °C unter Homogenisation (12.000 U/min mittels Ultra Turrax) abgekühlt und der eingangs beschriebenen Inprozesskontrolle unterzogen.

Die so erstellte Mischung wies eine homogene Dispersion mit gleichmäßig großen Teilchen auf.

Die entstandene Prädispersion wurde mittels Hochdruckhomogenisator feinstdispergiert. Es waren 3 Zyklen bei 800 bar erforderlich. Anschließend wurde das Gemisch unter Rühren und gleichmäßiger Homogenisation bei 9.000 U/min (Ultra Turrax) auf 35 °C abgekühlt. Das Vorphasengemisch wurde nun bei 350 bar (1 Zyklus) zwangshochdruckhomogenisiert und in einem separaten Gefäß bei 35 °C zwischengelagert.

Im Ansatzbehälter wurde nun die Phase 3 unter Rühren auf 50 °C erwärmt und so lange gerührt, bis die lipidischen Koimponenten vollständig geschmolzen waren. In einem weiteren Behälter wurde die Phase 6 ebenfalls unter Rühren auf 50 °C gebracht und so lange gerührt, bis eine klare Geldispersion entstanden war.

Die Phasen 4 und 5 wurde nun unter rühren zu Phase 3 gegeben und direkt anschließend Phase 6 zu dem Phasengemisch gegeben. Anschließend wurde bei 50 °C bei 18.000 U/min mittels Ultra Turrax 5 Minuten homogenisiert.

Anschließend wurden die Phasen 7, 8 und 9 zu der Mischung aus Phase 3+4+5+6 gegeben und bei 18.000 U/min mittels Ultra Turrax für 8 Minuten homogenisiert. Nun wurde die Dispersion aus den Phasen 3+4+5+6+7+8+9 bei 18.000 U/min mittels Ultra Turrax auf 45 °C abgekühlt. Nachfolgend wurde die Phase 10 zu der Mischung aus Phase 3+4+5+6+7+8+9 gegeben und während eines kontinuierlichen Homogenisierungsprozesses bei 18.000 U/min mittels Ultra Turrax auf die Zieltemperatur von 35°C homogenisiert. Dieser Vorgang dauerte 14 Minuten. Anschließend wurde die Prädispersion aus den Phasen 1+2 zu der Mischung aus den Phasen 3+4+5+6+7+8+9+10 gegeben und bei 18.000 U/min unter kontinuierlichem Rühren 18 Minuten homogenisiert. Abschließend wurde das Gemisch unter kontinuierlichem Homogenisieren bei 18.000 U/min auf 25 °C heruntergekühlt. Dieser Vorgang dauerte 11 Minuten.

### Zweite Wirksamkeitsstudie

Es wurde eine zweite Wirksamkeitsstudie als eine Capsaicin Empfindlichkeits-Kurzstudie an fünf Probanden durchgeführt, wobei ein herkömmliches, als Standardemulsion formuliertes Produkt C, ein erfindungsgemäß formuliertes Produkt D, bei der das hydrierte Phospholipid zumindest teilweise in einer ortho rhombischen lamellaren kristallinen Struktur vorlag und ein weiteres herkömmliches Produkt E mit hexagonaler lamellarer kristalliner Struktur des Phospholipids miteinander verglichen wurden. Alle drei Produkte C bis E wiesen eine identische Konzentration an 4-t-Butylcyclohexanol auf.

Für diese Kurzstudie wurden fünf Probanden (4 männlich, 1 weiblicher) mit einem Durchschnittsalter von 39 Jahren eingesetzt.

Zunächst wurden die Nasolabialfalte links und rechts bei jedem Probanden mit einer 2 %igen Natriumlaurylsulfat-Lösung gereinigt. Hierzu wurden jeweils 0,2 ml der 2 %igen Natriumlaurylsulfat-Lösung auf die links- und rechtsseitige Nasolabialfalte aufgetragen und leicht während 5 Sekunden pro Testfeld einmassiert. Anschließend wurden die Flächen unter fließendem lauwarmen Wasser (Temperatur 35 °C ± 2 °C) zwei Minuten lang vorsichtig gewaschen. Hierbei wurde darauf geachtet, daß die Waschlösung vollständig entfernt wurde. Nachfolgend wurden die entsprechend gekennzeichneten Nasolabialfaltenbereiche mit einem weichen, handelsüblichen Papiertuch durch vorsichtiges gleichmäßiges Abtupfen getrocknet. Nach weiteren 10 Minuten wurden jeweils 0,05 g der Produkte C und D (links und rechts der Nase) aufgetragen und leicht einmassiert.

Nach 8 bis 10 Minuten wurde per Pipette 0,02 g eines flüssigen Capsaicin-Extraktes auf alle Testfelder aufgetragen, wobei dieses käuflich zu erwerbende Capsaicin Flüssigextraktes 3 %ig in einem Verhältnis 1:10 mit Sonnenblumenöl verdünnt wurde.

Nach 3 Minuten beurteilte jeder Proband subjektiv, jedoch ohne Beeinflussung durch Mitprobanden, die Stärke des Brennens und Stechens, von Null = kein Brennen/Stechen, 1 = mildes Brennen/Stechen, 2 = mäßiges Brennen/Stechen und 3 = schmerzhaftes oder sehr unangenehmes Brennen/Stechen bedeuten.

Nach 4 Tagen wurde mit denselben fünf Probanden diese Kurzstudie wiederholt, wobei der einzige Unterschied zu dem zuvor beschriebenen ersten Teil der Kurzstudie bei der Wiederholung nicht die Produkte C und D sondern die Produkte D und E miteinander verglichen wurden.

Das Produkt C wies folgende Inhaltsstoffe auf und wurde wie folgt hergestellt:

### Inhaltsstoffe der Formulierung C

| Phase | Rohstoff | [%] |
|---|---|---|
| 1 | Emulgin SML 20 | 1,800 |
| 1 | Caprylic/Capric Triglyceride | 18,000 |
| 1 | Pentylene Glycol | 5,000 |
| 1 | Glycerin 99,5 % | 1,000 |
| 1 | Acrylates/Vinyl Isodecanoate Crosspolymer | 0,300 |
| 1 | Sodium Carbomer | 0,300 |
| 2 | Wasser | 72,400 |
| 3 | 4-T-Butylcyclohexanol, Pentylene Glycol (Symsitive) | 1,200 |
| | Σ | 100,000 |

Phase 1 wurde unter gleichmäßigem Rühren auf 25 °C erwärmt. Die Gelkörper wurden gleichmäßig in der Phase dispergiert. Anschließend wurde Phase 2 ebenfalls auf 25 °C erwärmt. Nun wurde Phase 2 zu der Phase 1 gegeben und anschließend bei 16.000 U/min mittels Ultra Turrax 5 Minuten homogenisiert und einer mikroskopischen Inprozesskontrolle unterzogen.

Die erstellte Mischung wies eine homogene Dispersion mit gleichmäßig kleinsten Teilchen auf.

Abschließend wurde die Phase 3 zu der Dispersion aus Phase 1+2 gegeben und bei 19.000 U/min mittels Ultra Turrax für 5 Minuten homogenisiert. Nach dieser Zeit war eine gleichmäßge Dispersion entstanden.

Das Produkt D wies folgende Inhaltsstoffe auf und wurde wie folgt hergestellt:

| Phase | Rohstoff | [%] |
|---|---|---|
| 1 | Hydriertes Phospholipid | 1,800 |
| 1 | Carnauba Wachs | 1,500 |
| 1 | Isostearly Isostearate | 3,500 |
| 1 | Pentylene Glycol | 1,500 |
| 1 | Glycerin 99,5 % | 1,000 |
| 2 | Wasser | 20,700 |
| 3 | Moringa Butter | 5,000 |
| 3 | Acrylates/Vinyl Isodecanoate Crosspolymer | 0,280 |
| 3 | Isostearly Isostearate | 8,000 |
| 4 | Xanthan Gum | 0,100 |
| 4 | Wasser | 46,720 |
| 4 | Pentylene Glycol | 0,750 |
| 4 | Hydroxyethylcellulose | 0,150 |
| 4 | Glycerin 99,5 % | 6,000 |
| 4 | 1,2 Hexandiol | 1,500 |
| 5 | Natronlauge 30 %ig | 0,300 |
| 6 | 4-T-Butylcyclohexanol, Pentylene Glycol (Symsitive) | 1,200 |
| | Σ | 100,000 |

Phase 1 wurde unter gleichmäßigem Rühren auf 85 °C erwärmt. Anschließend wurde Phase 2 ebenfalls auf 90 °C erwärmt. Bei 85 °C wurde Phase 2 zu der Phase 1 gegeben und anschließend bei 16.000 U/min mittels Ultra Turrax 5 Minuten homogenisiert. Anschließend wurde die Mischung auf 78 °C unter Homogenisation (14.000 U/min mittels Ultra Turrax) abgekühlt und einer mikroskopischen Inprozesskontrolle unterzogen.

Die so erstellte Mischung wies eine homogene Dispersion mit gleichmäßig großen Teilchen auf.

Die entstandene Prädispersion wurde mittels Hochdruckhomogenisator feinstdispergiert. Es waren 3 Zyklen bei 800 bar erforderlich. Anschließend wurde das Gemisch unter Rühren und gleichmäßiger Homogenisation bei 10.000 U/min (Ultra Turrax) auf 35 °C abgekühlt. Das Vorphasengemisch wurde nun bei 350 bar (1 Zyklus) zwangshochdruckhomogenisiert und in einem separaten Gefäß bei 35 °C zwischengelagert.

Im Ansatzbehälter wurde nun die Phase 3 unter Rühren auf 50 °C erwärmt und so lange gerührt, bis die Lipidkomponenten vollständig geschmolzen sind. In einem weiteren Behälter wurde unter Dispergieren die Phase 4 bei 50 °C gerührt, bis eine klare, gelbliche Geldispersion entstanden ist. Die Phase 4 wurde nun zu der Phase 3 gegeben und anschließend bei 85 °C bei 19.000 U/min mittels Ultra Turrax 5 Minuten homogenisiert.

Anschließend wurde die Phase 5 zu der Mischung aus Phase 3+4 gegeben und bei 19.000 U/min mittels Ultra Turrax für 5 Minuten homogenisiert. Nun wurde die Dispersion aus den Phasen 3+4+5 bei 18.000 U/min mittels Ultra Turrax auf 45 °C abgekühlt. Nachfolgend wurde die Phase 6 zu der Mischung aus Phase 3+4+5 gegeben und während eines kontinuierlichen Homogenisierungsprozesses bei 18.000 U/min mittels Ultra Turrax auf die Zieltemperatur von 35°C homogenisiert. Dieser Vorgang dauerte 16 Minuten. Anschließend wurde die Prädispersion aus den Phasen 1+2 zu der Mischung aus den Phasen 3+4+5+6 gegeben und bei 20.000 U/min unter kontinuierlichem Rühren 18 Minuten homogenisiert. Abschließend wurde das Gemisch unter kontinuierlichem Homogenisieren bei 13.000 U/min auf 25 °C heruntergekühlt. Dieser Vorgang dauerte 16 Minuten.

Das Produkt E wies folgende Inhaltsstoffe auf und wurde wie folgt hergestellt:

| Phase | Rohstoff | [%] |
|---|---|---|
| 1 | Hydriertes Phospholipid | 1,800 |
| 1 | Caprylic Capric Triglyceride | 5,000 |
| 1 | Pentylene Glycol | 1,500 |
| 1 | Glycerin 99,5 % | 1,000 |
| 2 | Wasser | 20,700 |
| 3 | Moringa Butter | 5,000 |
| 3 | Acrylates/Vinyl Isodecanoate Crosspolymer | 0,280 |
| 3 | Isostearly Isostearate | 8,000 |
| 4 | Xanthan Gum | 0,100 |
| 4 | Wasser | 46,720 |
| 4 | Pentylene Glycol | 0,750 |
| 4 | Hydroxyethylcellulose | 0,150 |
| 4 | Glycerin 99,5 % | 6,000 |
| 4 | 1,2 Hexandiol | 1,500 |
| 5 | Natronlauge 30 %ig | 0,300 |
| 6 | 4-T-Butylcyclohexanol, Pentylene Glycol (Symsitive) | 1,200 |
| | Σ | 100,000 |

Phase 1 wurde unter gleichmäßigem Rühren auf 85 °C erwärmt. Anschließend wurde Phase 2 ebenfalls auf 90 °C erwärmt. Bei 85 °C wurde Phase 2 zu der Phase 1 gegeben und anschließend bei 12.000 U/min mittels Ultra Turrax 5 Minuten homogenisiert. Anschließend wurde die Mischung auf 78 °C unter Homogenisation (12.000 U/min mittels Ultra Turrax) abgekühlt und der eingangs beschriebenen Inprozesskontrolle unterzogen.

Die so erstellte Mischung wies eine homogene Dispersion mit gleichmäßig großen Teilchen auf.

Die entstandene Prädispersion wurde mittels Hochdruckhomogenisator feinstdispergiert. Es waren 2 Zyklen bei 790 bar erforderlich. Anschließend wurde das Gemisch unter Rühren und gleichmäßiger Homogenisation bei 8.000 U/min (Ultra Turrax) auf 35 °C abgekühlt. Das Vorphasengemisch wurde nun bei 100 bar (1 Zyklus) zwangshochdruckhomogenisiert und in einem separaten Gefäß bei 35 °C zwischengelagert.

Im Ansatzbehälter wurde nun die Phase 3 unter Rühren auf 50 °C erwärmt und so lange gerührt, bis die Lipidkomponenten vollständig geschmolzen sind. In einem weiteren Behälter wurde unter Dispergieren die Phase 4 bei 50 °C gerührt, bis eine klare, gelbliche Geldispersion entstanden war. Die Phase 4 wurde nun zu der Phase 3 gegeben und anschließend bei 85 °C bei 19.000 U/min mittels Ultra Turrax 5 Minuten homogenisiert.

Anschließend wurde die Phase 5 zu der Mischung aus Phase 3+4 gegeben und bei 19.000 U/min mittels Ultra Turrax für 5 Minuten homogenisiert. Nun wurde die Dispersion aus den Phasen 3+4+5 bei 18.000 U/min mittels Ultra Turrax auf 45 °C abgekühlt. Nachfolgend wurde die Phase 6 zu der Mischung aus Phase 3+4+5 gegeben und während eines kontinuierlichen Homogenisierungsprozesses bei 18.000 U/min mittels Ultra Turrax auf die Zieltemperatur von 35°C homogenisiert. Dieser Vorgang dauerte 16 Minuten. Anschließend wurde die Prädispersion aus den Phasen 1+2 zu der Mischung aus den Phasen 3+4+5+6 gegeben und bei 16.000 U/min unter kontinuierlichem Rühren 14 Minuten homogenisiert. Abschließend wird das Gemisch unter kontinuierlichem Homogenisieren bei 14.000 U/min auf 25 °C heruntergekühlt. Dieser Vorgang dauerte 12 Minuten.

Das Ergebnis dieser zweiten Wirksamkeitsstudie ist nachfolgend in den Tabellen 2 und 3 zusammengefaßt.

**Tabelle 2:**

| | Produkt C | | Produkt D (ortho rhombisch) | |
|---|---|---|---|---|
| Proband | Brennen | Stechen | Brennen | Stechen |
| 1 | 2 | 3 | 1 | 1 |
| 2 | 2 | 3 | 1 | 1 |
| 3 | 3 | 3 | 2 | 2 |
| 4 | 2 | 3 | 1 | 1 |
| 5 | 3 | 3 | 2 | 1 |
| MW | 2,4 | 2,8 | 1,4 | 1,2 |

**Tabelle 3:**

| | Produkt D (ortho rhombisch) | | Produkt E (hexagonal) | |
|---|---|---|---|---|
| Proband | Brennen | Stechen | Brennen | Stechen |
| 1 | 1 | 1 | 2 | 1 |
| 2 | 1 | 2 | 2 | 2 |
| 3 | 2 | 2 | 2 | 2 |
| 4 | 1 | 1 | 2 | 1 |
| 5 | 1 | 1 | 2 | 2 |
| MW | 1,2 | 1,4 | 2,0 | 1,6 |

Von den Produkten D und E wurden die zuvor beschriebenen Röntgenstrukturuntersuchungen durchgeführt, mit dem Ergebnis, daß das Produkt D jeweils zwei Peaks bei 4,16 Ä und bei 3,71 Ä aufwies und somit eine ortho rhombisch lamellare kristalline Struktur enthält, während das Produkt E im Röntgendiagramm nur einen einzige Peak bei 4,16 Ä zeigte, und somit ausschließlich eine hexagonal lamellare kristalline Struktur enthält.

Die deutliche Überlegenheit der erfindungsgemäßen Formulierung wird auch durch diese zweite Wirksamkeitsstudie augenfällig belegt.

### Weitere Untersuchungen am Stratum Corneum, isoliert aus menschlicher Haut

Um den physiologischen Einfluß von Zusammensetzungen, die die bekannten hexagonalen lamellaren kristallinen Strukturen einerseits und die im vorliegenden Text vielfach beschriebenen ortho rhombischen lamellaren kristallinen Strukturen andererseits aufweisen, auf die geschädigte Haut im Hinblick auf deren Einfluß auf Strukturveränderungen zu untersuchen, wurde die nachfolgend beschriebene Studie durchgeführt. Hierzu wird in der Literatur, so insbesondere in der Veröffentlichung von J. C. Garson et al in J. Invest. Dermatol. 96:43-49, 1991, die Verwendung von delipidiertem Stratum Corneum empfohlen, da dieses dem in vivo Hautzustand am nächsten kommt.

Die Studie wurde mit einer Röntgenstreuungs Synchroton Mikro Strahlungsquelle ausgeführt, die eine Erfassung von Klein- und Weitwinkel Streuungsmustern an einem Stratum Corneum Muster mit einer räumlichen Auflösung von 1 Mikrometer erlaubt. Stratum corneum wurde isoliert aus Hautgewebe, gewonnen durch abdominale plastische Chirurgie, durch Separation von der Epidermis durch Immersion in 56°C warmem Wasser und anschließender tryptischer Digestion bei 40°C. Anschließend wurde ein Teil durch 6h Extraktion in Chloroform/ Methanol (2:1) von den interzellulären Stratum Corneum Lipiden entfettet.

Zwei nachfolgend noch näher beschriebene Produkte F und G wurden in einer Menge von 3 mg/cm² auf die externe Seite des in der vorstehend beschriebenen Weise behandelten Stratum corneum aufgetragen und 0,5 x 3 mm große Stücke in die Haltevorrichtung gespannt.

Die technischen Parameter bei dieser Röntgenstrukturuntersuchung waren unter Berücksichtigung der zuvor aufgeführten Veröffentlichung wie folgt:
- Experimente wurden durchgeführt an ESRF Beamline ID13
- Energie: 13,6keV, i.e. λ=0,9117 Å, mode 16 bunches
- Strahlengröße:cross-section 1.5(v) x 2(h)µm²
- Transmissionsgeometrie: Strahl parallel zur Oberfläche des Stratum Corneums
- Detektion: Frelon Camera, Pixel Grösse :50 x 50 µm²
- Abstand Muster-Detektor: 133,9 mm
- Bereich der Retikular Abstände: 15 bis 0,3 nm, z.B: für den Streu Vektor S(=1/d): 0,070 bis 3,3 nm⁻¹
- Expositionszeiten: 0,5 Sekunden
- Für jedes Muster wurden die Streuungsdaten entlang 3D Scans gesammelt, über die komplette Dicke des Stratum corneums (3 x 40 Positionen im Abstand von 2 µm), 3 Scans / Muster an 3 verschiedenen Positionen wurden durchgeführt, T = 22,5°C, relative Luftfeuchtigkeit 30%.

Daten Analyse
- rechtwinklige Integration entlang des Schmalwinkel Äquators (parallel zur Oberfläche des Stratum Corneums)
   - anguläre Integration (-20° bis +20°) entlang des Weitwinkel Meridians (perpendikular zur Oberfläche des Stratum Corneums)
   - Daten Analyse mit FIT2D Software

Die bei dieser Untersuchung verwendeten Produkte F und G wiesen folgende Inhaltsstoffe auf:

| | | erfindungsgemäße Formulierung Produkt F | herkömmliches Produkt G |
|---|---|---|---|
| Phase | Rohstoff | [%] | [%] |
| 1 | Hydriertes Phospholipid | 1,800 | 1,800 |
| 1 | Carnauba Wachs | 1,500 | 0,000 |
| 1 | Caprylic/Capric Triglyceride | 0,000 | 1,500 |
| 1 | Isostearly Isostearate | 3,500 | 3,500 |
| 1 | Pentylene Glycol | 1,500 | 1,500 |
| 1 | Glycerin 99,5 % | 1,000 | 1,000 |
| 2 | Wasser | 20,700 | 20,700 |
| 3 | Moringa Butter | 5,000 | 5,000 |
| 3 | Acrylates/Vinyl Isodecanoate Crosspolymer | 0,280 | 0,280 |
| 3 | Isostearly Isostearate | 8,000 | 8,000 |
| 4 | Xanthan Gum | 0,100 | 0,100 |
| 4 | Wasser | 46,720 | 46,720 |
| 4 | Pentylene Glycol | 0,750 | 0,750 |
| 4 | Hydroxyethylcellulose | 0,150 | 0,150 |
| 4 | Glycerin 99,5 % | 6,000 | 6,000 |
| 4 | 1,2 Hexandiol | 1,500 | 1,500 |
| 5 | Natronlauge 30 %ig | 0,300 | 0,300 |
| 6 | 4-T-Butylcyclohexanol, Pentylene Glycol (Symsitive) | 1,200 | 1,200 |
| | Σ | 100,000 | 100,000 |

Das Produkt F weist ortho rhombische lamellare kristalline Strukturen auf, während das herkömmliche Produkt G hexagonale lamellare kristalline Strukturen besitzt.

Das Produkt F wurde wie folgt hergestellt:
Phase 1 wurde unter gleichmäßigem Rühren auf 85 °C erwärmt. Anschließend wurde Phase 2 ebenfalls auf 90 °C erwärmt. Bei 85 °C wurde Phase 2 zu der Phase 1 gegeben und anschließend bei 16.000 U/min mittels Ultra Turrax 5 Minuten homogenisiert. Anschließend wurde die Mischung auf 78 °C unter Homogenisation (14.000 U/min mittels Ultra Turrax) abgekühlt und der eingangs beschriebenen Inprozesskontrolle unterzogen.

Die so erstellte Mischung wies eine homogene Dispersion mit gleichmäßig großen Teilchen auf.

Die entstandene Prädispersion wurde mittels Hochdruckhomogenisator feinstdispergiert. Es waren 3 Zyklen bei 800 bar erforderlich. Anschließend wurde das Gemisch unter Rühren und gleichmäßiger Homogenisation bei 10.000 U/min (Ultra Turrax) auf 35 °C abgekühlt. Das Vorphasengemisch wurde nun bei 350 bar (1 Zyklus) zwangshochdruckhomogenisiert und in einem separaten Gefäß bei 35 °C zwischengelagert.

Im Ansatzbehälter wurde nun die Phase 3 unter Rühren auf 50 °C erwärmt und so lange gerührt, bis die Lipidkomponenten vollständig geschmolzen waren. In einem weiteren Behälter wurde unter Dispergieren die Phase 4 bei 50 °C gerührt, bis eine klare, gelbliche Geldispersion entstanden war. Die Phase 4 wurde nun zu der Phase 3 gegeben und anschließend bei 85 °C bei 19.000 U/min mittels Ultra Turrax 5 Minuten homogenisiert.

Hiernach wurde die Phase 5 zu der Mischung aus Phase 3+4 gegeben und bei 19.000 U/min mittels Ultra Turrax für 5 Minuten homogenisiert. Nun wurde die Dispersion aus den Phasen 3+4+5 bei 18.000 U/min mittels Ultra Turrax auf 45 °C abgekühlt. Nachfolgend wurde die Phase 6 zu der Mischung aus Phase 3+4+5 gegeben und während eines kontinuierlichen Homogenisierungsprozesses bei 18.000 U/min mittels Ultra Turrax auf die Zieltemperatur von 35°C homogenisiert. Dieser Vorgang dauerte 16 Minuten. Anschließend wurde die Prädispersion aus den Phasen 1+2 zu der Mischung aus den Phasen 3+4+5+6 gegeben und bei 20.000 U/min unter kontinuierlichem Rühren 18 Minuten homogenisiert. Abschließend wurde das Gemisch unter kontinuierlichem Homogenisieren bei 18.000 U/min auf 25 °C heruntergekühlt. Dieser Vorgang dauerte 16 Minuten.

Das herkömmliche Produkt G wurde wie folgt hergestellt:
Phase 1 wurde unter gleichmäßigem Rühren auf 85 °C erwärmt. Anschließend wurde Phase 2 ebenfalls auf 90 °C erwärmt. Bei 85 °C wurde Phase 2 zu der Phase 1 gegeben und anschließend bei 12.000 U/min mittels Ultra Turrax 5 Minuten homogenisiert. Anschließend wurde die Mischung auf 78 °C unter Homogenisation (12.000 U/min mittels Ultra Turrax) abgekühlt und der eingangs beschriebenen Inprozesskontrolle unterzogen.

Die so erstellte Mischung wies eine homogene Dispersion mit gleichmäßig großen Teilchen auf.

Die entstandene Prädispersion wurde mittels Hochdruckhomogenisator feinstdispergiert. Es waren 2 Zyklen bei 790 bar erforderlich. Anschließend wurde das Gemisch unter Rühren und gleichmäßiger Homogenisation bei 8.000 U/min (Ultra Turrax) auf 35 °C abgekühlt. Das Vorphasengemisch wurde nun bei 100 bar (1 Zyklus) zwangshochdruckhomogenisiert und in einem separaten Gefäß bei 35 °C zwischengelagert.

Im Ansatzbehälter wurde nun die Phase 3 unter Rühren auf 50 °C erwärmt und so lange gerührt, bis die Lipidkomponenten vollständig geschmolzen waren. In einem weiteren Behälter wurde unter Dispergieren die Phase 4 bei 50 °C gerührt, bis eine klare, gelbliche Geldispersion entstanden war. Die Phase 4 wurde nun zu der Phase 3 gegeben und anschließend bei 85 °C bei 19.000 U/min mittels Ultra Turrax 5 Minuten homogenisiert.

Anschließend wurde die Phase 5 zu der Mischung aus Phase 3+4 gegeben und bei 19.000 U/min mittels Ultra Turrax für 5 Minuten homogenisiert. Nun wurde die Dispersion aus den Phasen 3+4+5 bei 18.000 U/min mittels Ultra Turrax auf 45 °C abgekühlt. Nachfolgend wurde die Phase 6 zu der Mischung aus Phase 3+4+5 gegeben und während eines kontinuierlichen Homogenisierungsprozesses bei 18.000 U/min mittels Ultra Turrax auf die Zieltemperatur von 35°C homogenisiert. Dieser Vorgang dauerte 16 Minuten. Anschließend wurde die Prädispersion aus den Phasen 1+2 zu der Mischung aus den Phasen 3+4+5+6 gegeben und bei 16.000 U/min unter kontinuierlichem Rühren 14 Minuten homogenisiert. Abschließend wurde das Gemisch unter kontinuierlichem Homogenisieren bei 14.000 U/min auf 25 °C heruntergekühlt. Dieser Vorgang dauerte 12 Minuten.

Als Ergebnis dieser Untersuchung ist festzuhalten, daß die mit der Röntgenweitwinkelstreuungsmessung detektierten Strukturen der beiden aufgetragenen Produkte F und G, d.h. die ortho rhombische lamellare kristalline Struktur des Produktes F und die hexagonale lamellare kristalline Struktur des Produktes G, jeweils auf der Oberfläche des behandelten Stratum corneums vorhanden ist.

Auf der mit dem Produkt F behandelten Oberfläche des Stratum corneums weist die Röntgenweitwinkelstreuungsmessung zwei scharfe Peaks bei 4,16 Ä und 3,71 Ä auf, was charakteristisch ist für die ortho rhombische lamellare kristalline Struktur des Produktes F, während die Röntgenweitwinkelstreuungsmessung der Oberfläche des mit dem Produkt G behandelten Stratum corneum nur einen einzigen Peak bei 4,16 Ä zeigt, was charakteristisch für die hexagonale lamellare kristalline Struktur des herkömmlichen Produktes G ist.

In tieferen Schichten des Stratum corneums verschwindet jedoch dieser Peak bei 4,16 Ä des mit dem Produkt G behandelten Stratum corneums, während sich in tieferen Schichten des mit dem erfindungsgemäßen Produkt F behandelten Stratum corneums beide Peaks bei 4,16 Ä und 3,71 Ä nachweisen lassen.

Hieraus ist zu schließen, daß in den tieferen Schichten des Stratum corneums die Kristallinität des herkömmlichen Produktes G vollständig verlorengeht, was zu einem Verlust der barriereunterstützenden Funktion führt, während das erfindungsgemäße Produkt F seine Kristallinität auch in tieferen Schichten des Stratum corneums beibehält und somit die für den Hautschutz und die Hautpflege erforderliche und erwünschte barriereunterstützende Funktion nicht nur auf der Oberfläche sondern auch in der Tiefe des Stratum corneums unverändert bewirkt.

## Patentansprüche

1. Kosmetisches Produkt, das neben Wasser mindestens ein hydriertes Phospholipid in einer Konzentration von wenigstens 0,7 Gew.%, mindestens einen zweiwertigen und/oder dreiwertigen Alkohol sowie mindestens ein pflanzliches Wachs enthält, **dadurch gekennzeichnet,**
a) **daß** das pflanzliche Wachs ein aus Blättern, Nadeln, Stengeln, Wurzeln, Rinden, Schalen, Samen, Blüten und/oder Früchten isoliertes Wachs ist,
b) **daß** in dem kosmetischen Produkt das Gewichtsverhältnis von hydriertem Phospholipid zu dem pflanzlichen Wachs zwischen 1:0,3 und 1:1,5, insbesondere zwischen 1:0,7 und 1:1, variiert,
c) **daß** in dem kosmetischen Produkt das hydrierte Phospholipid zumindest teilweise in einer ortho rhombischen lamellaren kristallinen Struktur vorliegt, und
d) **daß** das pflanzliche Wachs in der ortho rhombischen lamellaren kristallinen Struktur eingelagert und/oder an der ortho rhombischen lamellaren kristallinen Struktur angelagert ist.

2. Kosmetisches Produkt nach Anspruch 1, **dadurch gekennzeichnet, daß** das mindestens eine hydrierte Phospholipid aus der Gruppe ausgewählt ist, die hydriertes Phosphatidylethanolamin, hydriertes Phosphatidylinositol, hydriertes Phosphatidylcholin, hydriertes Lyso-Phosphatidylcholin, hydriertes Phosphatidylserin und hydrierte Phosphatidsäure umfaßt.

3. Kosmetisches Produkt nach Anspruch 1 oder 2, **dadurch gekennzeichnet, daß** das hydrierte Phospholipid ein hydriertes Phosphatidylcholin ist und eine Konzentration an hydriertem Phosphatidylcholin von wenigstens 60 Gew.% aufweist.

4. Kosmetisches Produkt nach einem der vorangehenden Ansprüche, **dadurch gekennzeichnet, daß** das pflanzliche Wachs aus der Gruppe ausgewählt ist, die Carnaubawachs, Candelillawachs, Ouricuriwachs, Zuckerrohrwachs, Retamowachs, Carandaywachs, Raffiawachs, Columbiawachs, Espartowachs, Alfalfawachs, Bambuswachs, Hanfwachs, Douglas Fir-Wachs, Korkwachs, Sisalwachs, Flachswachs, Baumwollwachs, Dammarwachs, Getreidewachs, Teewachs, Kaffeewachs, Ocatillawachs, Citrus Aurantium Dulcis Schalenwachs, Ficus Ceriferawachs, Orangenwachs, Sonnenblumenkernwachs, Sonnenblumenkernschalenwachs, Sprossenkohlwachs, Tabakpflanzenwachs, Kürbiskernwachs, Maiswachs, Feigenkaktus Wachs und Oleanderwachs umfaßt.

5. Kosmetisches Produkt nach einem der vorangehenden Ansprüche, **dadurch gekennzeichnet, daß** das Wachs insbesondere Carnaubawachs, Sonnenblumenkernwachs, Reiskleiewachs, Reiswachs oder eine Mischung dieser Wachse ist.

6. Kosmetisches Produkt nach Anspruch 4 oder 5, **dadurch gekennzeichnet, daß** der Carnaubawachs, Sonnenblumenkernwachs, Reiswachs und/oder das Reiskleiewachs als Hauptfettsäurekomponenten gesättigte C₂₂-C₂₆-Fettsäuren enthält.

7. Kosmetisches Produkt nach Anspruch 6, **dadurch gekennzeichnet, daß** das kosmetische Produkt ein solches Carnaubawachs, Sonnenblumenkernwachs, Reiswachs und/oder Reiskleiewachs aufweist, bei dem die Hauptfettsäurekomponente des jeweiligen Wachses in einer Konzentration zwischen 15 Gew.% und 33 Gew.%, bezogen auf das Gewicht des jeweiligen Wachses, vorhanden ist.

8. Kosmetisches Produkt nach Anspruch 7, **dadurch gekennzeichnet, daß** das kosmetische Produkt ein solches Wachs, insbesondere ein Carnaubawachs, Sonnenblumenkernwachs, Reiswachs und/oder ein Reiskleiewachs aufweist, bei dem die Hauptfettsäurekomponente des jeweiligen Wachses in einer Konzentration zwischen 23 Gew.% und 28 Gew.%, bezogen auf das Gewicht des jeweiligen Wachses, vorhanden ist.

9. Kosmetisches Produkt nach einem der Ansprüche 6 bis 8, **dadurch gekennzeichnet, daß** das Wachs und insbesondere das Carnaubawachs, Sonnenblumenkernwachs, Reiswachs und/oder das Reiskleiewachs freie C₁₆-C₂₄-Fettsäuren enthält.

10. Kosmetisches Produkt nach Anspruch 9, **dadurch gekennzeichnet, daß** das Wachs und insbesondere das Carnaubawachs, Sonnenblumenkernwachs, Reiswachs und/oder das Reiskleiewachs die freien C₁₆-C₂₄-Fettsäuren in einer Konzentration zwischen 1 Gew.% und 1,8 Gew.%, bezogen auf das Gewicht des jeweiligen Wachses, enthält.

11. Kosmetisches Produkt nach Anspruch 10, **dadurch gekennzeichnet, daß** die Konzentration der ungesättigten Fettsäuren in den freien Fettsäuren zwischen 0,05 Gew.% und 0,4 Gew.%, bezogen auf das Gewicht des jeweiligen Wachses variiert.

12. Kosmetisches Produkt nach einem der vorangehenden Ansprüche, **dadurch gekennzeichnet, daß** das kosmetische Produkt als zwei- oder dreiwertigen Alkohol ein Diol und/oder Glycerin enthält.

13. Kosmetisches Produkt nach einem der vorangehenden Ansprüche, **dadurch gekennzeichnet, daß** das kosmetische Produkt als Alkohol Glycerin, Pentylenglykol, bevorzugt 1,2-Pentandiol, 1,2-Hexandiol, Octandiol und/oder Butylcyclohexanol enthält.

14. Kosmetisches Produkt nach einem der vorangehenden Ansprüche, **dadurch gekennzeichnet, daß** das kosmetische Produkt als weiteren Inhaltsstoff Isostearylisostearat in einer Konzentration zwischen 0,5 Gew.% und 35 Gew.%, vorzugsweise zwischen 4 Gew.% und 20 Gew.%, enthält.

15. Kosmetisches Produkt nach einem der vorangehenden Ansprüche, **dadurch gekennzeichnet, daß** das kosmetische Produkt eine Konzentration an hydriertem Phosphatidylcholin zwischen 0,7 Gew.% und 8 Gew.%, insbesondere zwischen 1,2 Gew.% und 5 Gew.%, enthält.

16. Kosmetisches Produkt nach einem der vorangehenden Ansprüche, **dadurch gekennzeichnet, daß** das kosmetische Produkt desweiteren einen UV-Filter, einen hautschützenden Wirkstoff, einen hautpflegenden Wirkstoff, einen glättenden Wirkstoff, einen die Haut geschmeidigmachenden Wirkstoff, einen hautaufhellenden Wirkstoff, einen bräunenden Wirkstoff, einen desodorierenden Wirkstoff, einen enthaarenden Wirkstoff, einen feuchthaltenden Wirkstoff, einen Wirkstoff zur Pflege und Behandlung von überempfindlicher Haut, einen Wirkstoff zur Behandlung und Pflege infizierter, gereizter oder erkrankter Haut, einen Wirkstoff zur Prophylaxe gegen Insektenstiche, einen rückfettenden Wirkstoff, einen antientzündlichen Wirkstoff und/oder einen feuchtigkeitsspendenden Wirkstoff aufweist.

17. Kosmetisches Produkt nach einem der vorangehenden Ansprüche, **dadurch gekennzeichnet, daß** das kosmetische Produkt einen solchen Wirkstoff aufweist, der aus der Gruppe ausgewählt ist, die Butylcyclohexanol, Resolvin, Farnesyl Pyrophosphate, Capsazepine, Cinnamide, Carboxamide und Palmitoyl tripeptide-8 umfaßt.

18. Kosmetisches Produkt nach einem der vorangehenden Ansprüche, **dadurch gekennzeichnet, daß** das kosmetische Produkt als Butylcyclohexanol mindestens ein tertiäres Butylcyclohexanol und vorzugsweise ein 4-t-Butylcyclohexanol enthält.

19. Kosmetisches Produkt nach Anspruch 17 oder 18, **dadurch gekennzeichnet, daß** das kosmetische Produkt eine Mischung aus dem Butylcyclohexanol und Pentylenglycol aufweist.

20. Kosmetisches Produkt nach einem der Ansprüche 17 bis 19, **dadurch gekennzeichnet, daß** die Konzentration der zuvor aufgeführten Wirkstoffe in dem kosmetischen Produkt zwischen 0,01 Gew.% und 2,5 Gew.% variiert.

21. Konzentrat zur Herstellung des kosmetischen Produktes nach einem der Ansprüche 1 bis 20, **dadurch gekennzeichnet, daß** das kosmetische Produkt durch Verdünnung mit einem wäßrigen System herstellbar ist, wobei das Konzentrat mindestens ein hydriertes Phospholipid, Wasser, mindestens einen zwei- und/oder dreiwertigen Alkohol und mindestens ein pflanzliches Wachs enthält, wobei
a) das pflanzliche Wachs ein aus Blättern, Nadeln, Stengeln, Wurzeln, Rinden, Schaden, Samen, Blüten und/oder Früchten isoliertes Wachs ist,
b) das Konzentrat unter Ausbildung des kosmetischen Produktes in einem Volumenverhältnis zwischen 1:0,3 und 1:15 mit dem wäßrigen System verdünnbar ist, wobei
c) das Konzentrat eine solche Konzentration an hydriertem Phospholipid aufweist, daß abhängig von der erwünschten Verdünnung das hieraus durch Verdünnung hergestellte kosmetische Produkt mindestens 0,7 Gew.% des hydrierten Phospholipid enthält, wobei
d) im Konzentrat das Gewichtsverhältnis von hydriertem Phospholipid zu dem pflanzlichen Wachs zwischen 1:0,3 und 1:1,5, insbesondere zwischen 1:0,7 und 1:1, variiert, wobei
e) in dem Konzentrat das hydrierte Phospholipid zumindest teilweise in einer ortho rhombischen kristallinen lamellaren Struktur vorliegt, und wobei
f) in dem Konzentrat das pflanzliche Wachs in der ortho rhombischen lamellaren kristallinen Struktur eingelagert und/oder an der ortho rhombischen lamellaren kristallinen Struktur angelagert ist.

22. Konzentrat nach Anspruch 21, **dadurch gekennzeichnet, daß** das wäßrige System mindestens einen kosmetischen Wirkstoff aufweist.

## Claims

1. A cosmetic product which, in addition to water, contains at least one hydrogenated phospholipid in a concentration of at least 0.7% by weight, at least one divalent and/or trivalent alcohol and at least one vegetable wax, **characterized in that**
a) the vegetable wax is a wax isolated from leaves, needles, stems, roots, bark, skins, seeds, flowers and/or fruits,
b) that, in the cosmetic product, the weight ratio of hydrogenated phospholipid to vegetable wax varies between 1:0.3 and 1:1.5, in particular between 1:0.7 and 1:1,
c) that, in the cosmetic product, the hydrogenated phospholipid at least is in part in an ortho rhombic lamellar crystalline structure, and
d) that the vegetable wax is incorporated in the ortho rhombic lamellar crystalline structure and/or attached to the ortho rhombic lamellar crystalline structure.

2. The cosmetic product according to claim 1, **characterized in that** the at least one hydrogenated phospholipid is selected from the group consisting of hydrogenated phosphatidylethanolamine, hydrogenated phosphatidylinositol, hydrogenated phosphatidylcholine, hydrogenated lyso-phos-phatidylcholine, hydrogenated phosphatidylserine and hydrogenated phosphatidic acid.

3. The cosmetic product according to claim 1 or 2 **characterized in that** the hydrogenated phospholipid is a hydrogenated phosphatidylcholine and has a hydrogenated phosphatidylcholine concentration of at least 60% by weight.

4. The cosmetic product according to one of the preceding claims, **characterized in that** the vegetable wax is selected from the group consisting of carnauba wax, candelilla wax, ouricuri wax, sugar cane wax, retamo wax, caranday wax, raffia wax, columbia wax, esparto wax, alfalfa wax, bamboo wax, hemp wax, douglas fir wax, cork wax, sisal wax, flax wax, cotton wax, dammar wax, cereal wax, tea wax, coffee wax, ocatilla wax, citrus aurantium dulcis peel wax, ficus cerifera wax, orange wax, sunflower seed wax, sunflower seed shell wax, sprout cabbage wax, tobacco plant wax, pumpkin seed wax, corn wax, fig cactus wax and oleander wax.

5. The cosmetic product according to one of the preceding claims, **characterized in that** the wax is in particular carnauba wax, sunflower seed wax, rice bran wax, rice wax or a mixture of these waxes.

6. The cosmetic product according to claim 4 or 5, **characterized in that** the carnauba wax, sunflower seed wax, rice wax and/or the rice bran wax contain saturated C22-C26 fatty acids as main fatty acid components.

7. The cosmetic product according to claim 6, **characterized in that** the cosmetic product comprises such a carnauba wax, sunflower seed wax, rice wax and/or rice bran wax in which the main fatty acid component of the respective wax is present in a concentration between 15% and 33% by weight, based on the weight of the respective wax.

8. The cosmetic product according to claim 7, **characterized in that** the cosmetic product comprises such a wax, in particular a carnauba wax, sunflower seed wax, rice wax and/or a rice bran wax, in which the main fatty acid component of the respective wax is present in a concentration between 23 wt.% and 28 wt.%, based on the weight of the respective wax.

9. The cosmetic product according to one of claims 6 to 8, **characterized in that** the wax and in particular the carnauba wax, sunflower seed wax, rice wax and/or the rice bran wax contain free C16-C24 fatty acids.

10. The cosmetic product according to claim 9, **characterized in that** the wax and in particular the carnauba wax, sunflower seed wax, rice wax and/or the rice bran wax contain the free C16-C24 fatty acids in a concentration between 1 wt.% and 1.8 wt.%, based on the weight of the respective wax.

11. The cosmetic product according to claim 10, **characterized in that** the concentration of unsaturated fatty acids in the free fatty acids varies between 0.05% and 0.4% by weight relative to the weight of the respective wax.

12. The cosmetic product according to one of the preceding claims, **characterized in that** the cosmetic product contains a diol and/or glycerol as divalent or trivalent alcohol.

13. The cosmetic product according to one of the preceding claims, **characterized in that** the cosmetic product contains as alcohol glycerin, pentylene glycol, preferably 1,2-pentanediol, 1,2-hexanediol, octanediol and/or butylcyclohexanol.

14. The cosmetic product according to one of the preceding claims, **characterized in that** the cosmetic product contains as a further ingredient isostearylisostearate in a concentration between 0.5 wt.% and 35 wt.%, preferably between 4 wt.% and 20 wt.%.

15. The cosmetic product according to one of the preceding claims, **characterized in that** the cosmetic product contains a concentration of hydrogenated phosphatidylcholine between 0.7 wt.% and 8 wt.%, in particular between 1.2 wt.% and 5 wt.%.

16. The cosmetic product according to one of the preceding claims, **characterized in that** the cosmetic product further contains a UV filter, a skin-protecting active substance, a skin-caring active substance, a smoothing active substance, a skin-softening active substance, a skin-lightening active substance, a tanning active substance, a deodorizing active substance, comprises a depilatory active ingredient, a moisturizing active ingredient, an active ingredient for the care and treatment of hypersensitive skin, an active ingredient for the treatment and care of deficient, irritated or diseased skin, an active ingredient for the prophylaxis against insect bites, a refatting active ingredient, an antiinflammatory active ingredient and/or a moisturizing active ingredient.

17. The cosmetic product according to any of the foregoing claims, **characterised in that** the cosmetic product comprises an active ingredient selected from the group consisting of butylcyclohexanol, resolvin, farnesyl pyrophosphates, capsazepines, cinnamides, carboxamides and palmitoyl tripeptide-8.

18. The cosmetic product according to one of the preceding claims, **characterized in that** the cosmetic product contains, as butylcyclohexanol, at least one tertiary butylcyclohexanol and preferably a 4-t-butylcyclohexanol.

19. The cosmetic product according to claim 17 or 18, **characterized in that** the cosmetic product comprises a mixture of butylcyclohexanol and pentylene glycol.

20. The cosmetic product according to one of claims 17 to 19, **characterized in that** the concentration of the active substances listed above in the cosmetic product varies between 0.01 wt.% and 2.5 wt.%.

21. Concentrate for the preparation of the cosmetic product according to one of claims 1 to 20, **characterized in that** the cosmetic product can be prepared by dilution with an aqueous system, the concentrate containing at least one hydrogenated phospholipid, water, at least one divalent and/or trivalent alcohol and at least one vegetable wax, wherein
a) the vegetable wax is a wax isolated from leaves, needles, stems, roots, bark, damage, seeds, flowers and/or fruits,
b) the concentrate is dilutable with the aqueous system to form the cosmetic product in a volume ratio between 1:0.3 and 1:15 with the aqueous system, where
c) the concentrate has a concentration of hydrogenated phospholipid such that, depending on the desired dilution, the cosmetic product prepared therefrom by dilution contains at least 0.7% by weight of the hydrogenated phospholipid, where
d) in the concentrate, the weight ratio of hydrogenated phospholipid to the vegetable wax varies between 1:0.3 and 1:1.5, in particular between 1:0.7 and 1:1, wherein
e) in the concentrate, the hydrogenated phospholipid is at least partially present in an ortho rhombic crystalline lamellar structure, and wherein
f) in the concentrate the vegetable wax is incorporated in the ortho rhombic lamellar crystalline structure and/or attached to the ortho rhombic lamellar crystalline structure.

22. Concentrate according to claim 21, **characterized in that** the aqueous system comprises at least one cosmetic active ingredient.

## Revendications

1. Produit cosmétique, qui contient outre de l'eau au moins un phospholipide hydrogéné à une concentration d'au moins 0,7 % en poids, au moins un alcool bivalent et/ou trivalent, ainsi qu'au moins une cire végétale, **caractérisé en ce que**
a) la cire végétale est une cire isolée à partir de feuilles, d'aiguilles, de tiges, de racines, d'écorces, de peaux, de graines, de fleurs et/ou de fruits,
b) dans le produit cosmétique, le rapport pondéral du phospholipide hydrogéné sur la cire végétale varie entre 1:0,3 et 1:1,5, en particulier entre 1:0,7 et 1:1,
c) dans le produit cosmétique, le phospholipide hydrogéné est présent au moins en partie sous forme d'une structure cristalline lamellaire orthorhombique, et
d) la cire végétale est introduite dans la structure cristalline lamellaire orthorhombique et/ou est fixée sur la structure cristalline lamellaire orthorhombique.

2. Produit cosmétique selon la revendication 1, **caractérisé en ce que** l'au moins un phospholipide hydrogéné est choisi parmi le groupe qui comprend une phosphatidyléthanolamine hydrogénée, un phosphatidylinositol hydrogéné, une phosphatidylcholine hydrogénée, une lyso-phosphatidylcholine hydrogénée, une phosphatidylsérine hydrogénée et un acide phosphatidique hydrogéné.

3. Produit cosmétique selon la revendication 1 ou 2, **caractérisé en ce que** le phospholipide hydrogéné est une phosphatidylcholine hydrogénée et présente une concentration de phosphatidylcholine hydrogénée d'au moins 60 % en poids.

4. Produit cosmétique selon l'une quelconque des revendications précédentes, **caractérisé en ce que** la cire végétale est choisie parmi le groupe qui comprend la cire de carnauba, la cire de candelilla, la cire d'Ouricuri, la cire de canne à sucre, la cire de rétama, la cire de caranday, la cire de raphia, la cire de Colombie, la cire d'alfa, la cire d'alfalfa, la cire de bambou, la cire de chanvre, la cire de Douglas, la cire de liège, la cire de sisal, la cire de lin, la cire de coton, la cire de damar, la cire de blé, la cire de thé, la cire de café, la cire d'ocotillo, la cire de peaux de *Citrus Aurantium Dulcis*, la cire de *Ficus Cerifera*, la cire d'orange, la cire de graines de tournesol, la cire d'enveloppes de graines de tournesol, la cire de chou de Bruxelles, la cire de tabac, la cire de graines de courge, la cire de maïs, la cire de nopal et la cire de laurier-rose.

5. Produit cosmétique selon l'une quelconque des revendications précédentes, **caractérisé en ce que** la cire est en particulier de la cire de carnauba, de la cire de graines de tournesol, de la cire de son de riz, de la cire de riz ou un mélange de ces cires.

6. Produit cosmétique selon la revendication 4 ou 5, **caractérisé en ce que** la cire de carnauba, la cire de graines de tournesol, la cire de riz et/ou la cire de son de riz contient comme composantes d'acides gras principaux des acides gras saturés en C22 à C26.

7. Produit cosmétique selon la revendication 6, **caractérisé en ce que** le produit cosmétique présente une cire de carnauba, de graines de tournesol, de riz et/ou de son de riz telle que la composante d'acides gras principaux de la cire respective est présente en une concentration comprise entre 15 % en poids et 33 % en poids, par rapport au poids de la cire respective.

8. Produit cosmétique selon la revendication 7, **caractérisé en ce que** le produit cosmétique présente une cire, en particulier une cire de carnauba, une cire de graines de tournesol, une cire de riz et/ou une cire de son de riz, telle que la composante d'acides gras principaux de la cire respective est présente en une concentration comprise entre 23 % en poids et 28 % en poids, par rapport au poids de la cire respective.

9. Produit cosmétique selon l'une quelconque des revendications 6 à 8, **caractérisé en ce que** la cire, et en particulier la cire de carnauba, la cire de graines de tournesol, la cire de riz et/ou la cire de son de riz, contient des acides gras libres en C₁₆ à C₂₄.

10. Produit cosmétique selon la revendication 9, **caractérisé en ce que** la cire, et en particulier la cire de carnauba, la cire de graines de tournesol, la cire de riz et/ou la cire de son de riz, contient les acides gras libres en C₁₆ à C₂₄ en une concentration comprise entre 1 % en poids et 1,8 % en poids, par rapport au poids de la cire respective.

11. Produit cosmétique selon la revendication 10, **caractérisé en ce que** la concentration des acides gras insaturés dans les acides gras libres varie entre 0,05 % en poids et 0,4 % en poids, par rapport au poids de la cire respective.

12. Produit cosmétique selon l'une quelconque des revendications précédentes, **caractérisé en ce que** le produit cosmétique contient comme alcool bivalent ou trivalent un diol et/ou de la glycérine.

13. Produit cosmétique selon l'une quelconque des revendications précédentes, **caractérisé en ce que** le produit cosmétique contient comme alcool de la glycérine, du pentylène glycol, de préférence du pentane-1,2-diol, de l'hexane-1,2-diol, de l'octane-diol et/ou du butylcyclohexanol.

14. Produit cosmétique selon l'une quelconque des revendications précédentes, **caractérisé en ce que** le produit cosmétique contient comme autre ingrédient de l'isostéarylisostéarate en une concentration comprise entre 0,5 % en poids et 35 % en poids, de préférence entre 4 % en poids et 20 % en poids.

15. Produit cosmétique selon l'une quelconque des revendications précédentes, **caractérisé en ce que** le produit cosmétique contient une concentration de phosphatidylcholine hydrogénée comprise entre 0,7 % en poids et 8 % en poids, en particulier entre 1,2 % en poids et 5 % en poids.

16. Produit cosmétique selon l'une quelconque des revendications précédentes, **caractérisé en ce que** le produit cosmétique présente en outre un filtre UV, une substance dermoprotectrice, une substance de soin de la peau, une substance lissante, une substance rendant la peau plus souple, une substance éclaircissant la peau, une substance bronzante, une substance désodorisante, une substance dépilatoire, une substance préservant l'hydratation de la peau, une substance pour le soin et le traitement de peaux hypersensibles, une substance pour le traitement et le soin de peaux infectées, irritées ou malades, une substance pour la prophylaxie contre les piqûres d'insectes, une substance relipidante, une substance anti-inflammatoire et/ou une substance hydratante.

17. Produit cosmétique selon l'une quelconque des revendications précédentes, **caractérisé en ce que** le produit cosmétique présente une telle substance qui est choisie parmi le groupe qui comprend le butylcyclohexanol, la résolvine, le farnésyl-pyrophosphate, la capsazépine, le cinnamide, le carboxamide et le palmitoyl tripeptide-8.

18. Produit cosmétique selon l'une quelconque des revendications précédentes, **caractérisé en ce que** le produit cosmétique contient comme butylcyclohexanol au moins un butylcyclohexanol tertiaire et de préférence un 4-t-butylcyclohexanol.

19. Produit cosmétique selon la revendication 17 ou 18, **caractérisé en ce que** le produit cosmétique présente un mélange de butylcyclohexanol et de pentylène glycol.

20. Produit cosmétique selon l'une quelconque des revendications 17 à 19, **caractérisé en ce que** la concentration des substances susmentionnées dans le produit cosmétique varie entre 0,01 % en poids et 2,5 % en poids.

21. Concentré destiné à la fabrication du produit cosmétique selon l'une quelconque des revendications 1 à 20, **caractérisé en ce que** le produit cosmétique peut être fabriqué par dilution avec un système aqueux, dans lequel le concentré contient au moins un phospholipide hydrogéné, de l'eau, au moins un alcool bivalent et/ou trivalent et au moins une cire végétale, dans lequel
a) la cire végétale est une cire isolée à partir de feuilles, d'aiguilles, de tiges, de racines, d'écorces, de peaux, de graines, de fleurs et/ou de fruits,
b) le concentré pour former le produit cosmétique peut être dilué avec le système aqueux selon un rapport volumique compris entre 1:0,3 et 1:15, dans lequel
c) le concentré présente une concentration en phospholipide hydrogéné telle que, en fonction de la dilution souhaitée, le produit cosmétique fabriqué à partir de celui-ci par dilution contient au moins 0,7 % en poids du phospholipide hydrogéné, dans lequel
d) dans le concentré, le rapport pondéral du phospholipide hydrogéné sur la cire végétale varie entre 1:0,3 et 1:1,5, en particulier entre 1:0,7 et 1:1, dans lequel
e) dans le concentré, le phospholipide hydrogéné est présent au moins en partie sous forme d'une structure cristalline lamellaire orthorhombique, et dans lequel
d) dans le concentré, la cire végétale est introduite dans la structure cristalline lamellaire orthorhombique et/ou est fixée sur la structure cristalline lamellaire orthorhombique.

22. Concentré selon la revendication 21, **caractérisé en ce que** le système aqueux présente au moins une substance cosmétique.
